# EUROPÄISCHE PATENTSCHRIFT

(11) **EP 3 810 564 B1**
(45) Veröffentlichungstag und Bekanntmachung des Hinweises auf die Patenterteilung: **06.11.2024**
(21) Anmeldenummer: 19730834.9
(22) Anmeldetag: 19.06.2019
(51) Int. Cl.: C07C 5/48, C07C 11/04, C07C 51/215, C07C 53/08

(54) **VERFAHREN UND ANLAGE ZUR HERSTELLUNG EINES ODER MEHRERER OLEFINE UND EINER ODER MEHRERER CARBONSÄUREN**
METHOD AND INSTALLATION FOR THE PRODUCTION OF ONE OR MORE OLEFINS AND ONE OR MORE CARBOXYLIC ACIDS
PROCÉDÉ ET SYSTÈME DE PRODUCTION D'UNE OU D'UNE PLURALITÉ DE OLEFINS ET D'UN OU D'UNE PLURALITÉ D'ACIDES CARBOXYLIQUES

(30) Priorität: 21.06.2018 EP 18179086
(43) Veröffentlichungstag der Anmeldung: 28.04.2021
(73) Patentinhaber: Linde GmbH, 82049 Pullach (DE)
(72) Erfinder: ZELLHUBER, Mathieu, 82152 Martinsried (DE); SCHUBERT, Martin, 81375 München (DE); MEISWINKEL, Andreas, 83253 Rimsting (DE); WINKLER, Florian, 81241 München (DE); TOTA, Desislava, 81479 München (DE); ZANDER, Hans-Jörg, 81479 München (DE)
(86) Internationale Anmeldenummer: PCT/EP2019/066308
(87) Internationale Veröffentlichungsnummer: WO 2019/243480

(56) Entgegenhaltungen:
- EP-A1- 0 261 264
- WO-A1-2017/144584
- US-A1- 2010 256 432

## Beschreibung

Die Erfindung betrifft ein Verfahren zur Herstellung eines oder mehrerer Olefine und einer oder mehrerer Carbonsäuren sowie eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

In der WO 2017/144584 A1 ist ein Reaktor für die ODH offenbart, in dem zwei Reaktionszonen vorhanden sind. Es werden zwei getrennte Kühlmittelkreisläufe eingesetzt und in den Reaktionszonen sind unterschiedliche Katalysatoren vorhanden.

Aus der US 2010/256432 A1 ist ein Verfahren zur oxidativen Dehydrierung von Ethan bekannt. Ein Produktstrom der oxidativen Dehydrierung wird dabei mit einem Sauerstoffeliminierungskatalysator in einer Sauerstoffeliminierungsreaktionszone in Kontakt gebracht, um mindestens einen Teil des Sauerstoffs zu verbrennen. Anschließend erfolgt eine trenntechische Aufbereitung.

Die EP 0 261 264 A1 offenbart ein Verfahren zur Oxydehydrierung von Ethan zu Ethylen in einem Reationssystem mit offenen, in Reihe geschalteten Stufen, das die Änderung des Gesamtwasser- und Essigsäuregehalts im Ausgangsgasstrom nach mindestens einer anderen Stufe als der letzten Stufe der Reihe einschließt.

Bei der ODH werden insbesondere bei Verwendung von MoVNbTeOx-Katalysatoren unter industriell relevanten Reaktionsbedingungen signifikante Mengen der jeweiligen Carbonsäuren der eingesetzten Paraffine als Nebenprodukte gebildet. In diesem Zusammenhang wird ebenfalls auf einschlägige Fachliteratur wie Li, X. und Iglesia E., Kinetics and Mechanism of Ethane Oxidation to Acetic Acid on Catalysts Based on Mo-V-Nb Oxides, J. Phys. Chem.C, Bd. 112, 2008, Seiten 15001 bis 15008, verwiesen. Für einen wirtschaftlichen Anlagenbetrieb ist eine entsprechende Koppelproduktion von Olefinen und der jeweiligen Carbonsäuren bei Verwendung des beschriebenen Katalysatortyps in der Regel unausweichlich. Dies gilt insbesondere für die Herstellung von Ethylen durch ODH von Ethan (ODH-E), bei der gleichzeitig Essigsäure gebildet wird, aber auch für weitere, unten näher erläuterte Fälle.

In der industriellen Praxis gelten Koppelproduktionsverfahren im Allgemeinen als weniger interessant, da sie immer mit einer begrenzten Produktionsflexibilität einhergehen. Um ein solches Verfahren attraktiv zu gestalten, muss dem Betreiber eine einfach steuerbare, flexible Anlage zur Verfügung gestellt werden, um eine möglichst einfache Anpassung der Produktverteilung an den tatsächlichen und/oder wirtschaftlich sinnvollen Bedarf zu ermöglichen. In bestimmten Fällen kann es in entsprechenden Verfahren wünschenswert sein, die Produktverteilung in Richtung eines der gebildeten Produkte, bei der ODH-E beispielsweise in Richtung von Ethylen, zu verschieben, insbesondere wenn eine bessere Vermarktbarkeit (größeres Marktvolumen) für das jeweilige Produkt besteht. Ferner sind eine möglichst hohe Selektivität zu dem gewünschten Produkt sowie ein maximaler Umsatz der Edukte wünschenswert, um durch die geringeren zu bearbeitenden Gasvolumina Investitions- und Betriebskosten zu reduzieren. Die vorliegende Erfindung stellt sich diese Aufgabe.

### Offenbarung der Erfindung

Vor diesem Hintergrund schlägt die vorliegende Erfindung ein Verfahren zur Herstellung eines oder mehrerer Olefine und einer oder mehrerer Carbonsäuren sowie eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 95%, 96%, 97%, 98%, 99%, 99,5%, 99,9% oder 99,99% und die Angabe "arm" für einen Gehalt von höchstens 5%, 4%, 3%, 2%, 1%, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch um ein an der jeweiligen Komponente reiches Gemisch handeln.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte verwendet werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

### Vorteile der Erfindung

Wie erwähnt, ist für einen wirtschaftlichen Anlagenbetrieb eine Koppelproduktion von Ethylen und Essigsäure bei Verwendung des beschriebenen Katalysatortyps in der ODH, insbesondere der ODH-E, in der Regel unausweichlich, obwohl in der industriellen Praxis Koppelproduktionsverfahren im Allgemeinen als weniger attraktiv gelten. Die Ausgestaltung eines flexiblen, katalytischen Prozesses ist herausfordernd, insbesondere wenn es sich um einen exothermen Prozess wie die ODH, insbesondere die ODH-E, handelt. In diesem Fall muss stets die Gefahr eines thermischen Durchgangs unterbunden werden, was die Einstellung der Betriebsparameter teilweise stark einschränkt. Des Weiteren beinhalten die katalytischen Prozesse eine Vielzahl von Teilreaktionen, die sich wechselseitig beeinflussen. Es ist daher in der Regel sehr schwierig, geeignete Prozessgrößen zu identifizieren, die die Reaktion zuverlässig beschreiben und als Prozessleitgröße geeignet sind. Entsprechendes gilt für die Reaktorauslegung sowie für das Design des oder der verwendeten Katalysatoren.

Ist nachfolgend vereinfacht von einer Produktion von Ethylen und Essigsäure die Rede, schließt dies nicht aus, dass im Rahmen des erfindungsgemäßen Verfahrens auch höhere Olefine und Carbonsäuren gebildet werden können, insbesondere bei Verwendung von entsprechenden Einsätzen, die neben Ethan auch höhere Paraffine enthalten. Während beispielsweise beim Steamcracken auch aus schwereren Paraffinen leichtere Olefine gebildet werden können, beispielsweise aus Propan Ethylen, ist dies bei der ODH, insbesondere der ODH-E, nicht notwendigerweise der Fall. So wird beispielsweise Propan hier vorwiegend zu Propylen und Acrylsäure (Propensäure), nicht aber zu Ethylen umgesetzt. Es kann jedoch auch eine Weiterreaktion zu leichteren Produkten erfolgen, beispielsweise indem Acrylsäure durch Abspaltung von Kohlendioxid zu Ethylen umgesetzt wird, welches anschließend zu Essigsäure weiterreagiert. Eine entsprechende Reaktion ist beispielsweise bei Naumann d'Alnoncourt, L.-I. et al., Journal of Catalysis, Band 311, Seiten 369 bis 385, beschrieben. Ist hier von einer "Herstellung eines Olefins und einer Carbonsäure" die Rede, können das Olefin und die Carbonsäure die gleiche oder eine unterschiedliche Anzahl von Kohlenstoffatomen aufweisen, obwohl sie aus nur einem Edukt gebildet werden. Die vorliegende Erfindung schließt ebenfalls explizit nicht aus, dass mehrere unterschiedliche Olefine und/oder Carbonsäuren aus einem oder mehreren unterschiedlichen Edukten gebildet werden können.

Die in der ODH gebildeten Carbonsäuren werden typischerweise mit Wasser aus einem in der ODH gebildeten Prozessgasstrom abgetrennt. Werden Paraffine unterschiedlicher Kettenlängen eingesetzt, erhält man dabei eine wässrige Lösung unterschiedlicher Carbonsäuren. Ist dies, und die gleichzeitige Bildung höherer Olefine, nicht erwünscht, kann ein Reaktionseinsatz auch derart gebildet werden, dass er keine höheren Paraffine enthält, beispielsweise durch eine stromauf vorgesehene Abtrennung. Die vorliegende Erfindung eignet sich insbesondere zum Einsatz im Zusammenhang mit der ODH-E, aber auch zur Herstellung höherer Olefine und Carbonsäuren durch die ODH entsprechender längerkettiger (schwererer, höherer), insbesondere linearer, Paraffine.

In herkömmlichen Reaktoren realer Größe ist in der ODH-E eine praktische Limitierung des Ethanumsatzes, beispielsweise bei 40 bis 45%, festzustellen. Eine weitere Erhöhung des Umsatzes führt zu schnell ansteigenden Verlusten in Nebenprodukte wie Kohlenstoffoxide (COx) und somit auch zu einer erhöhten Gefahr eines thermischen Durchgehens. Gleichzeitig wurde festgestellt, dass das Produktverhältnis von Ethylen zu Essigsäure in der ODH-E vom Wasserpartialdruck in einem Prozessgas am Reaktoraustritt abhängt. Der Wasserpartialdruck hängt wiederum maßgeblich vom Wasseranteil im Reaktionseinsatz und vom Reaktionsumsatz ab. Eine gewünschte Erhöhung des Ethanumsatzes würde zu höherem Wasserpartialdruck am Reaktoraustritt und somit zwangsläufig zu einer Verschiebung der Produktverteilung in Richtung von Essigsäure führen. Zusätzlich wurde festgestellt, dass es für einen kontinuierlichen Betrieb eines Reaktors für die ODH-E erforderlich ist, eine Mindestwasserverdünnung im Reaktionseinsatz einzuhalten, da sonst eine nennenswerte zeitliche Abnahme der Aktivität und somit der Katalysatorleistung auftritt.

Die vorliegende Erfindung beruht nun auf der Erkenntnis, dass sich die erläuterten Probleme zumindest teilweise durch Verwendung eines Reaktors mit mehreren Reaktionszonen lösen lassen. In den mehreren Reaktionszonen wird im Rahmen der vorliegenden Erfindung eine Temperaturbeeinflussung in unterschiedlichem Umfang vorgenommen, und zwar dergestalt, dass in dem Reaktor insgesamt eine minimale Reaktionstemperatur aufrecht erhalten bzw. sichergestellt wird, dass die Reaktionstemperatur in Richtung des Reaktoraustritts nicht unter einen vorbestimmten Wert absinkt. Dies wird durch eine selektive Temperaturbeeinflussung, d.h. eine Temperaturbeeinflussung in unterschiedlichem Umfang, in den einzelnen Reaktionszonen erreicht. Unter einer "Temperaturbeeinflussung in unterschiedlichem Umfang" in den mehreren Reaktionszonen sei dabei im Rahmen der vorliegenden Erfindung verstanden, dass in zumindest einer der Reaktionszonen eine Temperaturbeeinflussung vorgenommen wird, die von einer Temperaturbeeinflussung in zumindest einer weiteren der Reaktionszonen abweicht.

Grundsätzlich kann eine "Temperaturbeeinflussung" dabei im Rahmen der vorliegenden Erfindung eine Erwärmung oder eine Kühlung einer entsprechenden Reaktionszone umfassen. Ein Umfang einer Erwärmung kann insbesondere durch die Katalysatorbeladung und/oder Katalysatoraktivität pro Raumeinheit in einer entsprechenden Reaktionszone eingestellt werden. Da sich bei einer höheren Katalysatorbeladung und/oder Katalysatoraktivität pro Raumeinheit die jeweils freiwerdende Wärme entsprechend erhöht (also eine Temperaturbeeinflussung in einem größeren Umfang erfolgt), kann durch eine höhere Katalysatorbeladung und/oder Katalysatoraktivität pro Raumeinheit die Reaktionstemperatur entsprechend erhöht werden. Eine Erhöhung der Reaktionstemperatur kann aber auch dadurch erfolgen, dass in einer Reaktionszone, in der eine höhere Reaktionstemperatur erhalten werden soll, eine geringere Kühlung oder eine stärkere Beheizung mittels eines entsprechenden Temperiermittels vorgenommen wird. Erfindungsgemäß werden dabei Katalysatoren vom gleichen Katalysatortyp, d.h. Katalysatoren mit derselben Grundrezeptur, verwendet, die in unterschiedlichen Konzentrationen oder Gehalten pro Raumeinheit bereitgestellt werden. Die vorliegende Erfindung kann aber auch beide Alternativen in sinnvoller Kombination miteinander umfassen.

Insbesondere kann die vorliegende Erfindung mit mehrlagigen Katalysatorbetten zum Einsatz kommen, die jeweils in einem oder mehreren Reaktionsrohren eines entsprechenden Reaktors bereitgestellt werden. Die vorliegende Erfindung eröffnet dabei im Gegensatz zur Verwendung eines einlagigen Katalysatorbetts bzw. eines Reaktors mit nur einer Reaktionszone weitere Spielräume für eine wirtschaftliche Optimierung entsprechender Prozesse. Die einfache Anwendung eines mehrlagigen Katalysatorbetts bzw. eines Reaktors mit entsprechenden Reaktionszonen reicht jedoch hierzu nicht notwendigerweise aus, da es ohne die Anwendung weiterer Maßnahmen aufgrund des höheren Wasserpartialdrucks zu einer Verschiebung der Produktverteilung hin zu Essigsäure kommen könnte.

Ein im Rahmen der vorliegenden Erfindung eingesetzter Reaktor kann insbesondere als Rohrreaktor ausgebildet sein, d.h. als ein Reaktor, der eine Vielzahl zumindest teilweise parallel verlaufender Reaktionsrohre aufweist. Hierbei durchläuft jedes der Reaktionsrohre die entsprechenden Reaktionszonen bzw. ist mit entsprechenden Reaktionszonen ausgebildet. In jedem der Reaktionsrohre kann dabei ein mehrlagiges Katalysatorbett ausgebildet sein und/oder jedes der mehreren Reaktionsrohre kann entlang seiner Länge in unterschiedlichem Umfang mittels einer Temperiereinrichtung einer abschnittsweise unterschiedlichen Temperierung unterworfen werden, so dass sich entlang der Reaktionsrohre in unterschiedlichem Umfang temperaturbeeinflusste Reaktionszonen ausbilden. Ist nachfolgend von einem "Rohrreaktor" die Rede, kann es sich hierbei insbesondere um einen bekannten Rohrbündelreaktor handeln. Die genannten Begriffe werden in diesem Fall synonym verwendet. Zum Aufbau und zur Betriebsweise von Rohrbündelreaktoren sei auf gängige Lehrbücher verwiesen.

Im Rahmen der vorliegenden Erfindung kann insgesamt trotz erhöhter Umsatzraten im Vergleich zum Betrieb eines Reaktors mit nur einer entsprechenden Reaktionszone eine Verschiebung der Wertproduktselektivität zu mehr Ethylen erreicht werden. Dies wird bei denselben Dampfverdünnungsraten im Reaktionseinsatz erreicht. Die Vorteile der vorliegenden Erfindung ergeben sich dabei daraus, dass in Richtung des Reaktoraustritts die Reaktionstemperatur über einen Wert angehoben werden kann, der oberhalb des Werts liegt, der sich bei kontinuierlicher bzw. konstanter Reaktorauslegung ergeben würde. Die Reaktionstemperatur muss am Reaktoreintritt herkömmlicherweise begrenzt werden, damit eine maximale Reaktionstemperatur nicht überschritten wird. Eine entsprechende Beschränkung erweist sich jedoch, wie erfindungsgemäß erkannt wurde, in den nachfolgenden Reaktionszonen als nicht vorteilhaft, da sie am Reaktoraustritt dazu führt, dass die minimale vorteilhafte Reaktionstemperatur unterschritten wird. Durch eine erfindungsgemäß vorgeschlagene abweichende Temperierung wird dagegen sichergestellt, dass diese minimale Reaktionstemperatur nicht unterschritten wird.

Ein besonderer Vorteil der vorliegenden Erfindung ist der, dass bei der Bereitstellung von Katalysatorbetten bzw. entsprechender Reaktionszonen, die unterschiedliche Katalysatorbeladungen und/oder Katalysatoraktivitäten pro Raumeinheit aufweisen, lediglich Schichten mit variabler Katalysatoraktivität verwendet werden müssen, d.h. es kann lediglich der Anteil an inertem Material in den Katalysatorpartikeln verändert, die Rezeptur des aktiven Katalysatormaterials selbst aber für alle Katalysatorbetten bzw. Reaktionszonen gleich gehalten werden. Auf diese Weise ist im Rahmen der vorliegenden Erfindung eine günstige Herstellung großer Mengen an Katalysator möglich, der in entsprechenden Katalysatorbetten bzw. Reaktionszonen lediglich in unterschiedlichem Umfang mit Inertmaterial "verdünnt" wird. Durch entsprechende Maßnahmen kann in den jeweiligen Reaktionszonen eine besonders einfache Temperaturbeeinflussung in unterschiedlichem Umfang erzielt werden.

Insgesamt schlägt die vorliegende Erfindung vor diesem Hintergrund ein Verfahren zur Herstellung eines oder mehrerer Olefine und einer oder mehrerer Carbonsäuren vor. Wie bereits erläutert, bezieht sich die vorliegende Erfindung insbesondere auf die ODH-E, also den Fall, dass es sich bei einem entsprechenden Olefin um Ethylen und bei einer entsprechenden Carbonsäure um Essigsäure handelt. Mit anderen Worten beträgt in diesem Fall die Anzahl von Kohlenstoffatomen jeweils zwei und es werden ein Olefin und eine Carbonsäure gebildet. Das Verfahren kann jedoch, wie erwähnt, auch zur Herstellung höherer Olefine eingesetzt werden, beispielsweise zur Herstellung von Propylen und Propensäure aus Propan, wobei die Anzahl von Kohlenstoffatomen drei beträgt. Die Anzahl von Kohlenstoffatomen kann im Rahmen der vorliegenden Erfindung jedoch auch bei vier oder ggf. fünf liegen. Der Schwerpunkt der vorliegenden Erfindung liegt jedoch bei der ODH-E und die Erfindung wird nachfolgend auch insbesondere unter Bezugnahme auf die ODH-E beschrieben.

In dem erfindungsgemäßen Verfahren wird oder werden ein oder mehrere Paraffine einer oxidativen Dehydrierung unterworfen. Grundlagen der oxidativen Dehydrierung wurden bereits eingangs erläutert. Die oxidative Dehydrierung wird im Rahmen der vorliegenden Erfindung, wie erwähnt, insbesondere in einem Rohrreaktor durchgeführt, welcher insbesondere eine Anzahl von Reaktionsrohren aufweist, die das entsprechende Gasgemisch längs durchströmt. Die Reaktionsrohre sind dabei insbesondere durch einen Mantelraum geführt, welcher von einem Temperiermittel durchströmt wird. Der Mantelraum kann in einer Ausgestaltung der vorliegenden Erfindung auch unterteilt sein, so dass die Reaktionsrohre abschnittsweise unterschiedlich temperiert werden können. Abschnitte der Reaktionsrohre bilden dabei jeweils eine Reaktionszone. In den Reaktionsrohren befindet sich jeweils eine Trägerstruktur zur Aufnahme eines Katalysatormaterials (also des aktiven Katalysators und inerter Verdünnungskomponenten, auch als "Katalysatorbett" bezeichnet).

Unter einem "Katalysatorbett" wird hier insbesondere eine in einem entsprechenden Reaktor bzw. einem Reaktionsrohr eines entsprechenden Reaktors an einer bestimmten Position eingebrachte Schüttung bezeichnet, die Inertmaterial und aktiven Katalysator umfasst. Einander entsprechende Bereiche unterschiedlicher Reaktionsrohre können dabei insbesondere abschnittsweise mit Katalysatorbetten identischer Eigenschaften ausgestattet werden. Dies kann auch derart verstanden werden, dass ein Katalysatorbett in diesem Fall auf unterschiedliche Reaktionsrohre verteilt ist. Die Verdünnung des aktiven Katalysatormaterials mit Inertmaterial erfolgt bevorzugt während der Produktion entsprechender Schüttkörper, die ein Katalysatorbett bilden, und kann dergestalt ausgeführt werden, dass verschiedene Schüttkörper mit unterschiedlichen Anteilen an aktivem Katalysatormaterial bereitgestellt werden. Ein Katalysatorbett mit einem vorgegebenen Aktivitätsniveau besteht in diesem Fall vollständig aus gleichen Schüttkörpern mit dem entsprechenden Anteil an aktivem Katalysatormaterial. In einer anderen Ausführung können verschiedene Reaktionszonen mit reduzierter katalytischer Aktivität auch durch physikalische Mischung von inerten Schüttkörpern und Schüttkörpern mit höherem Anteil an aktivem Katalysatormaterial bereitgestellt werden.

Im Rahmen der vorliegenden Erfindung wird für die oxidative Dehydrierung ein Reaktor mit mehreren Reaktionszonen verwendet, wobei ein Gasgemisch mit dem einen oder den mehreren Paraffinen nacheinander durch die Reaktionszonen geführt wird, und wobei zumindest zwei der mehreren Reaktionszonen einen Katalysator vom gleichen Katalysatortyp aufweisen und einer Temperaturbeeinflussung in unterschiedlichem Umfang unterworfen werden. Wie erwähnt, sind für eine derartige Temperaturbeeinflussung grundsätzlich zwei Wege gangbar, wobei erfindungsgemäß die Katalysatoren vom gleichen Katalysatortyp sind, die in unterschiedlichen Konzentrationen oder Gehalten pro Raumeinheit bereitgestellt werden.

Ist hier davon die Rede, dass zwei Reaktionszonen einen Katalysator "vom gleichen Katalysatortyp" aufweisen, sei hierunter verstanden, dass in den Reaktionszonen hinsichtlich ihrer Zusammensetzung bzw. Rezeptur identische Katalysatoren in gleicher oder (durch entsprechende Verdünnung mit Inertmaterial) unterschiedlicher Konzentration vorhanden sind. Insbesondere weisen die entsprechenden Zonen jeweils einen bzw. zwei identische MoVNbTeOx-Katalysatoren auf, die die ODH katalysieren.

Insbesondere kann für die oxidative Dehydrierung ein Reaktor verwendet werden, bei dem die mehreren Reaktionszonen als geschichtete Struktur aus mehreren Katalysatorbetten oder als von einander getrennte Reaktionszonen mit jeweils einem Katalysatorbett ausgebildet sind. Auch eine Ausbildung entsprechender Reaktionszonen in Form mehrlagiger Katalysatorschüttungen, die in diesem Fall mehrere Katalysatorbetten bilden, ist im Rahmen der vorliegenden Erfindung grundsätzlich möglich. Hierbei wird ein das erwähnte Paraffin enthaltendes Gasgemisch nacheinander durch die genannten Reaktionszonen geführt. In dieser Ausgestaltung der vorliegenden Erfindung wird dabei das Katalysatorbett einer zweiten der genannten Reaktionszonen, durch die das Gasgemisch geführt wird, nachdem es zuvor durch eine erste der Reaktionszonen geführt wurde, mit einer höheren Katalysatorbeladung und/oder Katalysatoraktivität pro Raumeinheit ausgebildet als das Katalysatorbett der ersten Reaktionszone.

Die erfindungsgemäß vorgeschlagene Lösung hat insbesondere den Vorteil, dass durch mehrere Reaktionszonen sowohl der Umsatz des eingesetzten Paraffins als auch die Selektivität zu dem entsprechenden Olefin gegenüber nur einer Reaktionszone deutlich gesteigert und somit ein ODH-E Verfahren deutlich wirtschaftlicher betrieben werden kann.

In einem von der Anmelderin eingesetzten Pilotreaktor wurden, wenn dieser mit einem nur einzonigen Bett betrieben wurde, maximale Ethanumsätze erreicht, die im einzonigen Fall nicht weiter steigerbar waren, da dies mit einem thermischen Durchgehen des Reaktors einhergegangen wäre. Im Falle eines mehrlagigen Katalysatorbetts wurden bei sonst gleichen Bedingungen hinsichtlich Raumgeschwindigkeit, Druck und Zusammensetzung des Reaktionseinsatzes, nochmals erhöhte Ethanumsätze erzielt, ohne dabei die Gefahr eines thermischen Durchgehens zu haben.

Die erfindungsgemäße Lösung umfasst, dass dadurch auch effektiv unterschiedliche (Reaktions-)Temperaturen in den unterschiedlichen Zonen vorliegen, wobei die unterschiedlichen Reaktionstemperaturen beispielsweise durch eine Steigerung der Katalysatoraktivität in Fließrichtung und/oder eine zonal unterschiedliche Kühlung/Temperierung des Reaktors erreicht werden kann.

Die vorliegende Erfindung sieht in dieser Ausgestaltung, mit anderen Worten, vor, eine Katalysatorbeladung und/oder Katalysatoraktivität in Richtung des Reaktoraustritts zu erhöhen und dem gegenüber in Richtung des Reaktoreintritts zu verringern. Die Katalysatorbeladung und/oder Katalysatoraktivität kann dabei insbesondere durch unterschiedliche Verdünnungsgrade mittels Inertmaterial eingestellt werden, wobei das aktive Katalysatormaterial in den unterschiedlichen Reaktionszonen insbesondere identisch sein kann. Die Katalysatorbeladung und/oder Katalysatoraktivität wird im Rahmen der vorliegenden Erfindung insbesondere von Zone zu Zone stufenweise erhöht, was im Gegensatz zu einer graduellen Erhöhung eine besonders einfache Bereitstellung des jeweiligen Katalysatorbetts durch Zumischung einer jeweils festen Menge an Inertmaterial oder Verwendung derselben Schüttungskörper ermöglicht. Entsprechende Maßnahmen können mit einer weiteren abgestuften Temperierung der Reaktionszonen kombiniert werden.

Durch die damit im Rahmen der soeben erläuterten Ausgestaltung der vorliegenden Erfindung vorgeschlagene Verwendung eines mehrlagigen Katalysatorbetts bzw. eines Reaktors mit entsprechenden Reaktionszonen kann eine Umsatzsteigerung von Ethan bzw. einem anderen Paraffin bei nur geringen Verlusten an Gesamtwertprodukten (hier definiert als die Summe des oder der Olefine und der Carbonsäure oder der Carbonsäuren, insbesondere von Ethylen und Essigsäure) erreicht werden. Im Rahmen der vorliegenden Erfindung wird dabei insbesondere eine Maximaltemperatur eingehalten bzw. durch die Wahl der Katalysatoraktivität bzw. Katalysatorbeladung dafür gesorgt, dass eine entsprechende Maximaltemperatur nicht überschritten wird. Entsprechende Vorteile können auch mittels einer unterschiedlichen Temperierung mittels geeigneter Temperiereinheiten oder mit einer Kombination entsprechender Maßnahmen erzielt werden.

Durch die in Strömungsrichtung ansteigende Katalysatorbeladung bzw. Katalysatoraktivität pro Raumeinheit oder eine abgestufte Temperierung kann im Rahmen einer entsprechenden Ausgestaltung der vorliegenden Erfindung, wie nachfolgend erläutert, erreicht werden, dass in derartigen Bereichen durch die Einhaltung einer Minimaltemperatur, die sich aufgrund der jeweils vorliegenden Katalysatorbeladung und/oder Katalysatoraktivität bzw. die sich jeweils einstellende Exothermie und/oder die jeweils vorgenommene Temperierung ergibt, eine übermäßige Carbonsäureproduktion vermieden werden.

Ein grundlegendes Merkmal der vorliegenden Erfindung ist, dass die Festlegung der einzelnen Katalysatorbeladungen bzw. Katalysatoraktivitäten sowie die Dimensionierung der Reaktionszonen bzw. ihrer Katalysatorbetten bzw. eine entsprechende abgestufte Temperierung jeweils derart vorgenommen wird, dass in keinem der Katalysatorbetten eine Prozessgastemperatur in unzulässiger Weise unterschritten wird.

Gemäß einem besonders vorteilhaften Aspekt der vorliegenden Erfindung werden daher eine minimale und eine maximale Reaktionstemperatur vorgegeben und die Temperaturbeeinflussung, also die Katalysatorbeladung und/oder die Katalysatoraktivität pro Raumeinheit und/oder eine entsprechende Temperierung in den Katalysatorbetten wird derart ausgebildet bzw. eine entsprechende abgestufte Temperierung wird derart vorgenommen, dass in keiner der Reaktionszonen an jeweils einer vorgegebenen Position die maximale Reaktionstemperatur überschritten und die minimale Reaktionstemperatur unterschritten wird.

Wie erwähnt, kann eine derartige Ausbildung der Katalysatorbetten bzw. der Reaktionszonen auch umfassen, eine entsprechende Dimensionierung der Katalysatorbetten bzw. Reaktionszonen vorzunehmen. Insbesondere wird im Rahmen der vorliegenden Erfindung in Richtung des Reaktoraustritts, wo die höchsten Partialdrücke des oder der Olefine und die niedrigsten Partialdrücke des oder der Paraffine erreicht werden, eine entsprechend erhöhte Katalysatorbeladung und/oder Katalysatoraktivität vorgenommen bzw. bereitgestellt, wodurch sichergestellt werden kann, dass hier die minimale vorgegebene Reaktionstemperatur nicht unterschritten wird. Da in Richtung des Reaktoraustritts die Partialdrücke des oder der Paraffine deutlich niedriger sind als am Anfang, wird vorteilhafterweise auch deshalb eine höhere Katalysatoraktivität bereitgestellt, damit die "restlichen" Paraffine noch in ausreichender Menge umgesetzt (und damit auch die erforderliche Wärme für die Mindesttemperatur generiert) werden können.

Im Rahmen der vorliegenden Erfindung wird, wie mehrfach erwähnt, vorteilhafterweise ein Reaktor verwendet, der eine Anzahl zumindest teilweise parallel verlaufender Reaktionsrohre verwendet wird. Es handelt sich dabei also um einen Rohreaktor grundsätzlich bekannter Art bzw. einen Rohrbündelreaktor. Es ist insbesondere vorgesehen, dass die vorgegebene Position, an der die maximale Reaktionstemperatur nicht überschritten und die minimale Reaktionstemperatur nicht unterschritten werden soll, auf einer Mittelachse zumindest eines der mehreren Reaktionsrohre liegt.

Es kann jedoch im Rahmen der vorliegenden Erfindung auch vorgesehen sein, in gewissem Umfang eine Überschreitung und Unterschreitung entsprechender Temperaturgrenzen zuzulassen. So kann beispielsweise vorgesehen sein, dass das Verfahren derart durchgeführt wird, dass in mindestens 30%, 60%, 80%, 90%, 95% oder 99% einer jeden der Reaktionszonen die maximale Reaktionstemperatur nicht überschritten und die minimale Reaktionstemperatur nicht unterschritten wird. Hierbei können auch insbesondere erhöhte Mindestanforderungen in Richtung des Reaktoraustritts definiert werden. Mit anderen Worten kann ein entsprechendes Verfahren derart durchgeführt werden, dass in der zweiten Reaktionszone in einem höheren Prozentanteil des Katalysatorbetts die minimale Reaktionstemperatur nicht unterschritten wird als in dem Katalysatorbett der ersten Reaktionszone.

Die Vorteile der vorliegenden Erfindung ergeben sich insbesondere daraus, dass die Bildung von Essigsäure aus Ethylen (bzw. anderer Carbonsäuren ausgehend von entsprechenden Olefinen), ungeachtet möglicher zwischenzeitlicher De- und Adsorptionsschritte nach der Bildung von Ethylen bzw. des Olefins, eine bedeutend niedrigere Aktivierungsenergie und somit eine deutlich geringere Temperaturabhängigkeit als die weiteren Hauptreaktionen bei der ODH aufweist. Dies gilt insbesondere im Vergleich zur Bildung von Ethylen bzw. eines entsprechenden anderen Olefins ausgehend von Ethan bzw. des entsprechenden Paraffins, aber auch im Vergleich zu den verschiedenen Reaktionen, die zur Bildung von Kohlenstoffoxiden, also unerwünschten Nebenprodukten führen.

Die Quantifizierung der Aktivierungsenergien wurde im Rahmen der vorliegenden Erfindung auf Grundlage von Laborversuchen mit unterschiedlichen Einsatzzusammensetzungen vorgenommen. Diese Beobachtung des Katalysatorverhaltens ist insbesondere bemerkenswert, da bei erhöhten Temperaturen die Bildung von sämtlichen höher oxidierten Produkten wie Essigsäure, Kohlenmonoxid und Kohlendioxid begünstigt werden sollte. Beim Studium der Reaktionen in ODH-E-Reaktoren, die unter industriellen Bedingungen betrieben werden, konnte jedoch durch die Anmelderin gezeigt werden, dass die Bildungsreaktionen von Kohlenmonoxid und Kohlendioxid von höheren Temperaturen gegenüber der Bildungsreaktion von Essigsäure überproportional begünstigt werden. Grundsätzlich nehmen die Reaktionsgeschwindigkeiten aller Reaktionen, d.h. hier die Bildungsraten aller Produkte, mit einer Temperaturerhöhung zu. Der deutliche Unterschied in den Aktivierungsenergien, insbesondere die deutlich niedrigere Aktivierungsenergie der Folgereaktion Ethylen zu Essigsäure (und somit die deutlich geringere Temperaturabhängigkeit dieser Reaktion) gegenüber allen anderen Reaktionen bewirkt jedoch, dass die Weiterreaktion des Ethylens bzw. die Bildungsrate der Essigsäure durch die Weiterreaktion des Ethylens, gegenüber den anderen Reaktionen (Haupt-, Neben- und Folgereaktionen) in geringerem Maße erhöht wird. Dies bewirkt die beobachtete Selektivitätsverschiebung. Es sei jedoch betont, dass der geschilderte Mechanismus nicht notwendigerweise den experimentell beobachteten Effekten zugrunde liegen muss und die Erfindung daher durch die soeben getroffenen Erläuterungen nicht eingeschränkt wird.

Der Fachmann hätte, ausgehend von grundsätzlichen Überlegungen und ohne diese erfindungsgemäße überraschende Erkenntnis, es nicht für notwendig erachtet, eine bestimmte Minimaltemperatur in einer Reaktionszone bzw. in einem Katalysatorbett einzuhalten, da er bei zunehmender Temperatur von der Bildung in gleichem Maße zunehmender Mengen an Essigsäure ausgegangen wäre. Wie im Rahmen der vorliegenden Erfindung gezeigt werden konnte, ist jedoch das Gegenteil der Fall. Die Tatsache, dass gerade die Bildung von Essigsäure, die im Rahmen der vorliegenden Erfindung nicht erwünscht ist, bei geringeren Temperaturen vergleichsweise verstärkt erfolgt, ist überraschend. Der Fachmann wäre davon ausgegangen, dass sich die Bildung von Essigsäure bei erhöhter Temperatur in ähnlicher Weise verstärkt und hätte daher nicht einen Reaktor mit einer Ausgestaltung, wie sie erfindungsgemäß vorgeschlagen wird, gewählt bzw. betrieben. Er wäre daher bei einer entsprechend einfacheren Betriebsweise bzw. Reaktorgestaltung geblieben.

Die vorliegende Erfindung nutzt die stark unterschiedlichen Temperaturabhängigkeiten der einzelnen Reaktionen bei der ODH, um durch eine gezielte Beeinflussung der Temperaturbedingungen nicht nur den Umsatz und die Gesamtselektivität zu Wertprodukten gezielt zu steuern, sondern auch die Selektivitätsverteilung zwischen diesen Wertprodukten.

Im Rahmen der vorliegenden Erfindung wird insbesondere ein Rohrreaktor verwendet, der derart ausgestaltet ist, dass er eine Eintrittsöffnung und eine Austrittsöffnung aufweist, wobei mindestens zwei der erwähnten Reaktionszonen vorgesehen und zwischen der Eintrittsöffnung und der Austrittsöffnung des Reaktors angeordnet sind. Hierbei wird eine der Reaktionszonen, die näher an der Austrittsöffnung angeordnet ist, als eine andere der Reaktionszonen, mit einer erhöhten Katalysatorbeladung und/oder Katalysatoraktivität pro Raumeinheit ausgestattet oder in einem geringeren Umfang gekühlt als die andere der Reaktionszonen. Mit anderen Worten wird also reaktoraustrittsseitig im Rahmen der vorliegenden Erfindung eine erhöhte Katalysatorbeladung und/oder Katalysatoraktivität pro Raumeinheit gewählt oder eine geringere Kühlung vorgenommen. Die erhöhte Katalysatoraktivität oder eine geringere Kühlung kann im Rahmen der Erfindung insbesondere auch nur in der "letzten" Reaktionszone bzw. einem entsprechenden Katalysatorbett vorgenommen werden und die zuvor angeordneten Katalysatorbetten bzw. entsprechenden Reaktionszonen können geringere, insbesondere graduell geringere, Katalysatoraktivitäten und/oder Katalysatorbeladungen pro Raumeinheit aufweisen oder entsprechend stärker gekühlt werden. Die Katalysatoraktivitäten können, wie erwähnt, stufenweise von Zone zu Zone in Richtung des Reaktoraustritts ansteigen. Entsprechendes gilt im Fall einer im Rahmen der Erfindung vorgenommenen Temperierung.

Insbesondere kann vorgesehen sein, dass der Reaktor wenigstens eine weitere Reaktionszone aufweist, durch die das Gasgemisch geführt wird, bevor es durch die erste Reaktionszone und die zweite Reaktionszone geführt wird. Hierbei ist insbesondere vorgesehen, die zweite Reaktionszone mit einer höheren Katalysatorbeladung und/oder Katalysatoraktivität pro Raumeinheit als das Katalysatorbett der ersten Reaktionszone auszubilden bzw. eine nochmals geringere Kühlung vorzunehmen. Wie erwähnt, kann die weitere Reaktionszone auch eine geringere Katalysatorbeladung und/oder Katalysatoraktivität pro Raumeinheit als die erste Reaktionszone bzw. deren Katalysatorbett aufweisen.

Die im Rahmen der vorliegenden Erfindung einsetzbaren Katalysatoren wurden bereits zuvor erwähnt. Insbesondere können im Rahmen der vorliegenden Erfindung in sämtlichen Katalysatorbetten bzw. Reaktionszonen dieselben Katalysatoren bzw. Katalysatoren mit derselben Grundrezeptur verwendet werden. Diese können in unterschiedlichen Konzentrationen bzw. Gehalten pro Raumeinheit bereitgestellt werden, wobei eine Verdünnung, wie zuvor erwähnt, vorgenommen werden kann. Insbesondere können dabei sämtliche Reaktionszonen bzw. deren Katalysatorbetten jeweils einen Anteil am aktiven Katalysator von mindesten 0,1 Gewichtsprozent aufweisen. Der Gehalt an aktivem Katalysator kann auch beispielsweise mehr als 1, mehr als 5 oder mehr als 10 Gewichtsprozent aktivem Katalysatoranteils betragen. Der jeweilige Gehalt richtet sich nach der Aktivität des Katalysators. Wird eine unterschiedliche Temperierung der einzelnen Reaktionszonen vorgenommen, kann ggf. der Katalysator über die gesamte Länge der Reaktionsrohre auch völlig gleich gehalten werden. Beliebige Kombinationen sind möglich.

Im Rahmen der vorliegenden Erfindung ist insbesondere vorgesehen, dass die Reaktionszonen mittels eines Temperiersystems unter Einsatz eines oder mehrerer Temperiermittelströme temperiert werden. Hierbei kann insbesondere ein Temperiersystem mit unterschiedlichen Temperiermittelströmen, die selektiv bestimmte Reaktionszonen bzw. Katalysatorbetten temperieren, zum Einsatz kommen. Auf diese Weise kann eine besonders gezielte Anpassung an die jeweils erforderlichen Maximal- und Minimaltemperaturen erzielt werden. Es kann also insbesondere zumindest einer der Temperiermittelströme zur Temperierung nur einer oder nur eines Teils der Reaktionszonen verwendet werden. Eine "Temperierung" erfolgt insbesondere in Form einer Kühlung. Diese kann insbesondere mittels Flüssigsalz vorgenommen werden. Hierbei kann insbesondere in Richtung des Reaktoraustritts eine zunehmend geringere Kühlung vorgenommen werden.

Die vorliegende Erfindung beruht auch auf der überraschenden Erkenntnis, dass bei einem Wasserpartialdruck am Austritt eines oder mehrerer für die ODH-E verwendeter Reaktoren im Bereich von 0,5 bis 5 bar (abs.), insbesondere von 0,7 bis 3 bar (abs.), das Molenstromverhältnis von Essigsäure zu Ethylen im Austrittsstrom (nachfolgend überwiegend als "Prozessgas" bezeichnet) beinahe linear zum Wasserpartialdruck am Austritt verläuft. Dieser Wert lässt sich daher als Prozessleitgröße verwenden, wenn ein bestimmtes Produktverhältnis von Essigsäure zu Ethylen eingestellt werden soll. Der Wasserpartialdruck im Prozessgas ist dabei das Ergebnis sowohl der Wasserzugabe am Reaktoreintritt bzw. in einem entsprechenden Reaktionseinsatz als auch der Umsetzung des Ethans im Reaktor und damit ggf. auch der momentanen Katalysatoraktivität. Im Gegensatz zu einer Einstellung lediglich des Wassergehalts im Reaktionseinsatz, der ohne Kenntnis der genannten weiteren Einflussgrößen zu stark schwankenden Wasserpartialdrücken im Prozessgas und damit schwankenden Produktverhältnissen führen kann, kann durch die Verwendung des Wasserpartialdrucks im Prozessgas als Prozessleitgröße daher eine sehr viel exaktere Einstellung des gewünschten Produktverhältnisses erzielt werden. Gleichzeitig kann im Rahmen der vorliegenden Erfindung durch die Verwendung einer Mindestwassermenge im Reaktionseinsatz eine konstante Katalysatoraktivität aufrecht erhalten werden, die anderenfalls über die Zeit abnehmen würde.

Eine Einstellung des Wassergehalts im Reaktionseinsatz, nicht aber im Prozessgas, ist in der EP 1 201 630 A2 beschrieben. Ferner ist hier auch angegeben, dass Druck, Temperatur und Verweildauer in der Reaktionszone kontrolliert werden können. Wie hoch der Wassergehalt im Prozessgas ist, ist allerdings hier nicht thematisiert. Entsprechendes gilt auch für ein in der US 4,899,003 A beschriebenes Verfahren. Es fehlt damit in beiden Fällen an der Erkenntnis, dass der Wasserpartialdruck am Reaktoraustritt eine Prozessleitgröße darstellt, über die sich die Produktselektivität eines Verfahrens, in dem eine Koppelproduktion von Ethylen und Essigsäure mittels ODH-E unter Verwendung des erwähnten Katalysatortyps vorgenommen wird, besonders zuverlässig einstellen lässt.

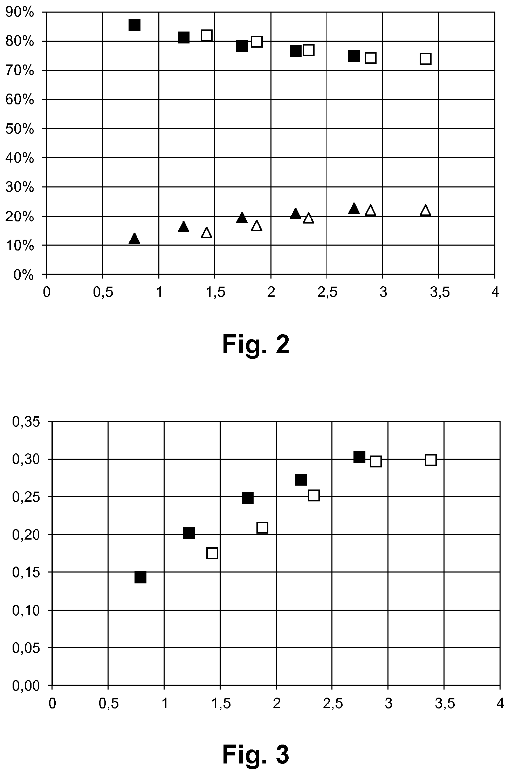

Die genannten Gesetzmäßigkeiten wurden zunächst im Rahmen von Ethanoxidationsversuchsreihen mit konstanter Eintrittstemperatur und variierendem Wasseranteil im Reaktionseinsatz unter Verwendung eines MoVNbTeOx-Katalysators festgestellt. Dabei konnte eine nahezu konstante Konversion des Ethans erreicht werden, bei ebenfalls nahezu konstanter Selektivität zu Kohlendioxid und Kohlenmonoxid. Die molaren Mengen an den gewünschten Produkten Ethylen und Essigsäure entwickelten sich hingegen in genau diesem Bereich konträr gegeneinander. In dem genannten Bereich zeigt sich ein stetiger, beinahe linearer gegensätzlicher Verlauf des Produktmolenstromverhältnisses von Essigsäure zu Ethylen. Zur weiteren Erläuterung wird auf die beigefügten Figuren 2 und 3 und die zugehörigen Erläuterungen verwiesen.

Es wurden zusätzlich analoge Versuchsreihen bei unterschiedlichen Durchströmraten und somit unterschiedlicher Raumgeschwindigkeit (Weight Hourly Space Velocity, WHSV) und Temperatur im Reaktor durchgeführt. Bei höherer Durchströmrate und somit höherer Raumgeschwindigkeit und niedrigerer Temperatur sind erwartungsgemäß niedrigere Konversionsraten zu beobachten, jedoch ist das Verhältnis der beiden Produktmolenströme bei gleichen Wasserpartialdrücken am Reaktoraustritt nahezu identisch mit den bei niedrigerer Durchströmrate bestimmten Werten. Dies zeigt, dass die Prozesssteuerung in dem genannten Bereich maßgeblich auf den Wasserpartialdruck am Austritt gestützt werden kann. Der teilweise klar lineare Verlauf des Produktmolenstromverhältnisses wird vor allem für den wirtschaftlich relevanten Betrieb bei höheren Konversionen ersichtlich.

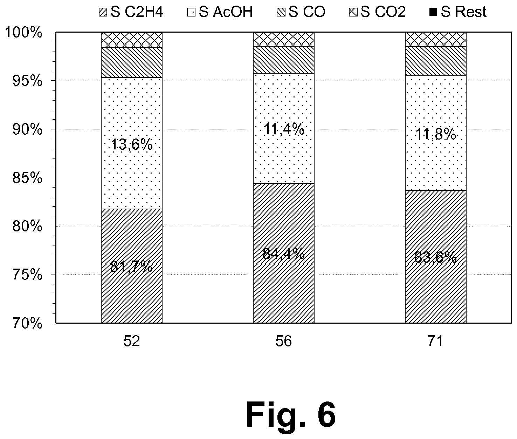

Weitere Versuchsreihen wurden unter Verwendung eines Versuchsreaktors durchgeführt, wobei ebenfalls die oben erwähnten Zusammenhänge belegt werden konnten. Zu Details sei insbesondere auf die beigefügte Figur 6 sowie die zugehörigen Erläuterungen verwiesen.

Die vorliegende Erfindung schlägt daher in einer besonders vorteilhaften Ausgestaltung vor, dass dem Reaktor ein wasserhaltiges Prozessgas entnommen wird und dass in dem Prozessgas ein Wasserpartialdruck, insbesondere in Abhängigkeit von einem vorgegebenen Produktverhältnis, insbesondere einem vorgegebenen Produktmolenstromverhältnis, von der Essigsäure zu dem Ethylen bzw. einer anderen Carbonsäure zu dem entsprechenden Olefin, auf einen Wert in einem Bereich zwischen 0,5 bis 5 bar (abs.), insbesondere in einem Bereich zwischen 0,7 und 3 bar (abs.), eingestellt wird. Wie erwähnt, ergibt sich in dem Bereich für unterschiedliche Konversionen und Betriebsbedingungen ein gleichbleibend stetiges, nahezu lineares Produktmolenstromverhältnis von Essigsäure zu Ethylen bzw. den erwähnten anderen Verbindungen, so dass hier eine besonders gut steuerbare Koppelproduktion dieser Verbindungen mit einstellbarem Produktionsschwerpunkt möglich ist.

Im Rahmen der vorliegenden Erfindung kann insgesamt trotz erhöhter Umsatzraten im Vergleich zum Betrieb mit einem einlagigen Katalysatorbett bzw. einem Reaktor mit nur einer entsprechenden Reaktionszone eine Verschiebung der Wertproduktselektivität zu mehr Ethylen erreicht werden. Dies wird bei denselben Dampfverdünnungsraten im Reaktionseinsatz erreicht. Die beschriebenen Maßnahmen zur Steuerung der zeitlichen Entwicklung der Katalysatoraktivität durch die Einstellung von unterschiedlichen Wasserpartialdrücken in dem dem Reaktor entnommenen Gasgemisch behalten auch bei Verwendung eines mehrlagigen Betts ihre Gültigkeit und sind insbesondere in Kombination vorteilhaft.

Die charakteristischen Selektivitätskurven können somit bei Verwendung eines adäquat ausgestalteten, mehrlagigen Katalysatorbetts bzw. einem Reaktor mit mehreren entsprechender Reaktionszonen parallel hin zu mehr Ethylen verschoben werden. Die Anpassungsmöglichkeiten im Betrieb auf Basis der Steuerung des Wasserpartialdrucks am Reaktoraustritt bleibt somit erhalten. Entsprechendes gilt auch für den Fall einer zonal unterschiedlichen Temperierung.

Die bei Verwendung eines einlagigen Betts beschriebenen Limitierungen in der weiteren wirtschaftlichen Optimierung des Prozesses können somit durch Anwendung einer Prozessführung mit mehrlagigen Betten und gezielter Temperatursteuerung überwunden werden. Die Wirtschaftlichkeit und die Vermarktungsfähigkeit der ODH- bzw. ODH-E-Technologie verbessern sich somit spürbar.

Im Rahmen der vorliegenden Erfindung wird der oxidativen Dehydrierung ein Gasgemisch unterworfen, das neben dem oder den Paraffinen auch Sauerstoff und insbesondere Verdünnungsmittel umfasst. Dieses Gasgemisch kann insbesondere auch in Form separater Stoffströme dem oder den verwendeten Reaktoren zugespeist und damit erst in dem oder den Reaktoren gebildet werden. Beispielsweise können ein paraffinhaltiger Stoffstrom und ein sauerstoffhaltiger Stoffstrom zu einem entsprechenden Reaktionseinsatz in dem oder den verwendeten Reaktoren oder stromauf des oder der Reaktoren vereinigt werden.

Das Gasgemisch oder eine oder mehrere Komponenten hiervon kann bzw. können jegliche verfahrenstechnische Behandlung wie Verdichtung, Entspannung, Abkühlung oder Erwärmung oder auch die Abtrennung von Teilströmen, die Zuspeisung weiterer Stoffströme oder eine chemische Umsetzung von Komponenten erfahren. Insbesondere umfasst im Rahmen der vorliegenden Erfindung das Bilden eines entsprechenden Gasgemischs beispielsweise eine Erwärmung. Bei dieser Erwärmung, der sogenannten Feedvorwärmung, kann das Gasgemisch auf eine Temperatur gebracht werden, die die ODH in einer sich anschließenden Reaktionseinheit mit einem oder mehreren Reaktoren anlaufen lässt.

Insbesondere kann in einem Verfahren gemäß einer Ausführungsform der Erfindung vorgesehen sein, dass das Bilden des Gasgemischs umfasst, einen Stoffstrom mit einem oder mehreren weiteren Fluiden zu vereinigen. Auf diese Weise können geeignete Medien zugespeist werden, die beispielsweise die Reaktionsbedingungen bei der ODH günstig beeinflussen. Wie erwähnt, handelt es sich bei der ODH um eine stark exotherme Reaktion, so dass typischerweise sogenannte Verdünnungsmittel wie Inertgase oder Dampf zugegeben werden, um ein thermisches Durchgehen zu verhindern. Entsprechende Verdünnungsmittel können bei der Bildung des Gasgemischs zugegeben werden, also stromauf oder erst in einem oder mehreren Reaktoren. Auch kann beispielsweise bereits bei der Bildung des Gasgemischs Sauerstoff oder ein sauerstoffhaltiges Gasgemisch zugegeben werden, der bei der ODH benötigt wird. Wahlweise erfolgt auch dies erst später.

Im Rahmen der vorliegenden Erfindung wird vorteilhafterweise der Wasserpartialdruck gemessen und es wird eine Regelung verwendet, mittels derer der Wasserpartialdruck unter Verwendung wenigstens einer Stellgröße eingestellt wird. Wie erwähnt, kann durch eine Regelung auf Grundlage des Wasserpartialdrucks eine sehr viel genauere Einstellung des Produktverhältnisses erzielt werden, als wenn lediglich eine Wasserzugabe im Reaktionseinsatz gesteuert würde.

Wie erwähnt kommt die vorliegende Erfindung insbesondere dann zum Einsatz, wenn in der oxidativen Dehydrierung ein Katalysator, der zumindest die Elemente Molybdän, Vanadium, Niob und optional Tellur enthält, also ein sogenannter MoVTeNbO-Katalysator, verwendet wird, weil sich bei Verwendung eines derartigen Katalysators Ethylen und Essigsäure bilden und die erwähnten Gesetzmäßigkeiten einstellen.

Die oxidative Dehydrierung wird im Rahmen der vorliegenden Erfindung vorteilhafterweise mit einer Paraffinkonversion von mindestens 15% durchgeführt. Die Ethankonversion kann dabei insbesondere bei mindestens 20, 25, 30, 35, 40 oder 45% liegen. Die Paraffinkonversion liegt insbesondere unterhalb von 75%. Das vorgegebene Produktmolenstromverhältnis von Essigsäure zu Ethylen bzw. einer anderen Carbonsäure zu einem anderen Olefin liegt dabei insbesondere in einem Bereich von 0,05 bis 0,5.

Unter der "Konversion" bzw. dem "Umsatz" wird dabei hier der molare Anteil der eingesetzten Edukte, hier des Ethans oder eines anderen Paraffins, verstanden, der insgesamt zu (Haupt- und Neben-) Produkten reagiert. Der "Produktmolenstrom" einer Komponente beschreibt die Molmenge einer Komponente, die pro Zeiteinheit aus einem oder mehreren Reaktoren austritt.

Der Wasserpartialdruck in dem Prozessgas kann im Rahmen der vorliegenden Erfindung insbesondere durch Zugabe von Wasser zu dem Reaktionseinsatzstrom und/oder durch Einstellen einer Reaktortemperatur, bei der die oxidative Dehydrierung durchgeführt wird, eingestellt werden. In diesem Zusammenhang kann insbesondere die zonal unterschiedliche Temperaturbeeinflussung, wie sie die vorliegende Erfindung vorschlägt, zum Einsatz kommen. Hierbei handelt es sich also um geeignete Stellgrößen der erwähnten Regelung. Es kann beispielsweise auch vorgesehen sein, mittels einer Wasserzugabe zu dem dem Reaktor zugeführten Gasgemisch eine Grob- und mittels der Einstellung einer Reaktortemperatur eine Feineinstellung vorzunehmen. Bei höherer Reaktortemperatur ergibt sich ein höherer Umsatz und damit eine höhere Bildung von Reaktionswasser. Hierbei wird der Wasserpartialdruck in dem Prozessgas also zumindest zum Teil durch Einstellen der Reaktortemperatur eingestellt.

Als eine weitere besonders maßgebliche Einflussgröße ergibt sich die zugegebene Menge an Sauerstoff im Reaktionseinsatz. Im Rahmen der vorliegenden Erfindung wird in der besonders vorteilhaften Ausgestaltung dieser Parameter stets so angepasst, dass am Reaktoraustritt ein Sauerstoffgehalt im Prozessgas zwischen 0,01 mol-% und 50 mol-%, bevorzugt zwischen 0,1 und 5 mol-%, besonders bevorzugt zwischen 0,1 und 0,5 mol-% stets eingehalten wird, um einerseits eine Reduktion des Katalysatormaterials durch Sauerstoffmangel zu vermeiden und andererseits sicherheitstechnische Risiken aufgrund hoher Sauerstoffgehalte zu limitieren. Aus diesen Einschränkungen ergibt sich jedoch der Umstand, dass die Regelung der Sauerstoffzudosierung der grundlegenden Festlegung des Betriebspunktes nachgeschaltet ist und keinen nennenswerten Einfluss auf das Produktmolenstromverhältnis besitzt, solange sichergestellt ist, dass der oben genannte Bereich für den Sauerstoffgehalt am Austritt eingehalten wird.

Im Rahmen der vorliegenden Erfindung wird als der einzustellende Wasserpartialdruck der Partialdruck an einem Reaktoraustritt eines oder mehrerer für die oxidative Dehydrierung verwendeter Reaktoren verstanden, beispielsweise direkt am Ende eines Katalysatorbetts oder einer mit diesem verbundenen Leitung. Insbesondere ist ein Prozessgas aus der oxidativen Dehydrierung am Reaktoraustritt noch keinen seine Zusammensetzung ändernden Maßnahmen, insbesondere einer Kühlung, einer Wäsche und dergleichen, unterworfen worden.

Besonders vorteilhaft ist es, wenn der Wasserpartialdruck an dem Reaktoraustritt des oder der Reaktoren erfasst und als Eingangsgröße einer Regelung verwendet wird. Verfahren zur Wasserbestimmung und damit zur Bestimmung des Wasserpartialdrucks sind dem Fachmann grundsätzlich bekannt. Beispielsweise kann es sich um gängige Absorptionsspektroskopiemethoden, z.B. Fourier-transformierte Infrarotspektroskopie (FTIR) oder Tunable-Diode-Laser-Absorptionsspektroskopie (TDLAS), in Kombination mit gängigen Druckmessmethoden handeln.

Mit besonderem Vorteil wird die oxidative Dehydrierung im Rahmen der vorliegenden Erfindung in einem Temperaturbereich bzw. auf einem Temperaturniveau von 240 bis 500 °C in einem Reaktorbett des oder der verwendeten Reaktoren durchgeführt. Insbesondere kann der Temperaturbereich bei 260 und 400 °C, besonders bevorzugt bei 280 bis 350 °C liegen. Der Gesamtdruck am Reaktoreintritt des oder der Reaktoren liegt vorzugsweise zwischen 1 und 10 bar (abs.), insbesondere zwischen 2 und 9 bar (abs.), weiter insbesondere zwischen 3 und 8 bar (abs.). Die Raumgeschwindigkeit im Reaktorbett des oder der Reaktoren (WHSV) liegt im Bereich zwischen 0,1 und 10 kg Paraffin/(h × kg Katalysator), bevorzugt zwischen 0,5 und 5 kg Paraffin/(h × kg Katalysator), besonders bevorzugt zwischen 0,7 und 3 kg Paraffin/(h × kg Katalysator). Insbesondere in diesem Bereich ist die zuvor erläuterte Einstellbarkeit der Produktmolenströme möglich.

Das erfindungsgemäße Verfahren kann insbesondere unter Verwendung eines oder mehrerer, dem Reaktionseinsatz zugegebener und in das Prozessgas übergehender Verdünnungsmittel durchgeführt werden. Die Verwendung entsprechender Verdünnungsmittel, die insbesondere sicherstellen, dass bei der stark exothermen ODH ein stabiler und sicherer Reaktorbetrieb gewährleistet wird, ist grundsätzlich bekannt. Wie erwähnt, kann zur Einstellung des gewünschten Wasserpartialdrucks in dem genannten Bereich insbesondere eine Zugabe von Wasser bzw. Wasserdampf in den Reaktionseinsatz erfolgen. Dieses Wasser bzw. dieser Wasserdampf wirkt zugleich als Verdünnungsmittel. Alternativ oder zusätzlich können aber ein oder mehrere weitere Verdünnungsmittel verwendet werden.

Insbesondere können im Rahmen der vorliegenden Erfindung ein oder mehrere Verdünnungsmittel zum Einsatz kommen, das oder die aus der Gruppe ausgewählt ist oder sind, die aus Wasser, Methan, Stickstoff und wenigstens einem weiteren Inertgas besteht. Auch Kohlendioxid kann als Verdünnungsmittel zum Einsatz kommen. Entsprechende Verdünnungsmittel nehmen an der Reaktion in dem oder den Reaktoren nicht oder allenfalls zu einem geringen Anteil teil und gehen daher zumindest zum überwiegenden Teil in das Prozessgas über.

Im Rahmen der vorliegenden Erfindung wurde ferner erkannt, dass im Falle der ODH-E auch bei Einbringung von Ethylen als zusätzlichem Feedstrom in den Reaktor, d.h. als Teil des Reaktionseinsatzes, ein starker funktionaler Zusammenhang zwischen dem Produktmolenstromverhältnis von Ethylen und Essigsäure und dem Wasserpartialdruck am Reaktoraustritt besteht. Die beschriebene Anlagenfahrweise kann somit auch bei zusätzlicher Ethyleneinspeisung angewandt werden. Dies ermöglicht es beispielsweise, die Flexibilität hin zu mehr Essigsäure als Produkt zu verstärken, falls dies erwünscht sein sollte. Dies führt allerdings dann zu erwartenden höheren Verlusten zu Kohlenmonoxid und Kohlendioxid. In bestimmten Fällen kann also eine Verfahrensvariante vorteilhaft sein, bei der dem Reaktionseinsatz ferner Ethylen in einer vorgegebenen Menge, insbesondere von 0 bis 50 Molprozent, zugegeben wird. Entsprechendes gilt für andere Olefine.

Die Einbringung von zusätzlichem Ethylen kann sowohl in Form einer Einspeisung aus einer externen Quelle als auch in Form einer Rückführung einer entsprechenden Fraktion aus dem Zerlegungsteil der Anlage selbst erfolgen. Bei dem "Zerlegungsteil" handelt es sich um eine Anordnung, in der mittels thermischer Trennung Komponenten oder Komponentengruppen aus dem Prozessgas oder einem aus diesem erhaltenen Gasgemisch abgetrennt werden. Diese Rückführung kann durch zusätzliche Entnahme einer entsprechenden Fraktion im Zerlegungsteil oder durch Änderung der Sumpfproduktspezifikation in einer Rektifikationskolonne erfolgen, die zur Trennung von Ethan und Ethylen verwendet wird, und die im Zerlegungsteil vorgesehen ist. In diesem Fall wird durch Anpassung der Trennbedingungen wie Kopftemperatur oder Druck, oder aber durch Verwendung einer entsprechend ausgebildeten, "unschärfer" trennenden Rektifikationskolonne gezielt ein Teil des ansonsten über Kopf abgezogenen Produkts Ethylen in den Sumpf der Rektifikationskolonne überführt und dort in einer ansonsten überwiegend Ethan enthaltenden Fraktion abgezogen. Diese kann in den oder die Reaktoren zurückgeführt werden.

Die vorliegende Erfindung erstreckt sich ferner auf eine Anlage zur Herstellung eines oder mehrerer Olefine und einer oder mehrerer Carbonsäuren. Zu weiteren Merkmalen und Vorteilen einer entsprechenden Anlage sei auf den entsprechenden unabhängigen Patentanspruch und die obigen Erläuterungen ausdrücklich verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

Kurze Beschreibung der Zeichnungen
Figur 1 veranschaulicht eine Anlage zur Herstellung von Ethylen und Essigsäure mit einem Reaktor gemäß einer Ausführungsform der Erfindung.
Figur 2 veranschaulicht Selektivitäten zu Ethylen und Essigsäure.
Figur 3 zeigt Produktmolenstromverhältnisse bezüglich Ethylen und Essigsäure zur Veranschaulichung des Hintergrunds der Erfindung.
Figur 4 zeigt Produktmolenstromverhältnisse bezüglich Ethylen und Essigsäure zur Veranschaulichung des Hintergrunds der Erfindung.
Figur 5 veranschaulicht ein Verfahren, das im Rahmen einer Ausgestaltung der vorliegenden Erfindung zum Einsatz kommen kann.
Figur 6 veranschaulicht Produktselektivitäten im Rahmen eines nicht erfindungsgemäßen Verfahrens.
Figur 7 veranschaulicht Produktselektivitäten im Rahmen eines nicht erfindungsgemäßen Verfahrens und im Rahmen eines Verfahrens gemäß einer Ausführungsform der Erfindung.
Figur 8 veranschaulicht Reaktortemperaturverläufe im Rahmen eines nicht erfindungsgemäßen Verfahrens und im Rahmen eines Verfahrens gemäß einer Ausführungsform der Erfindung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

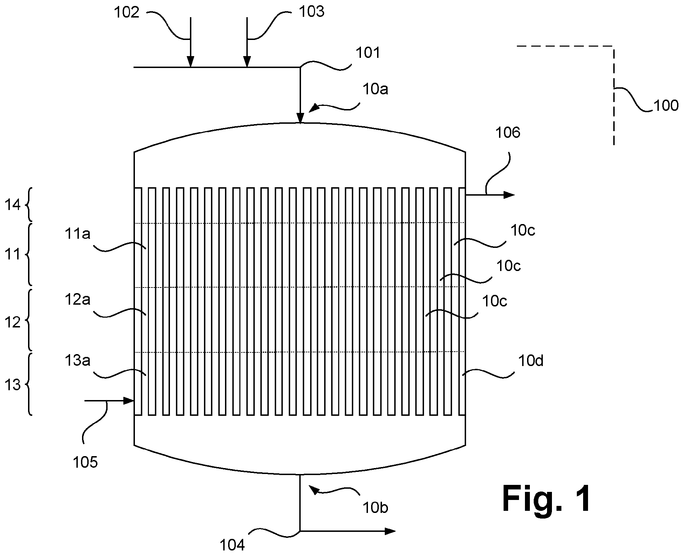

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Die Anlage 100 ist dabei nur schematisch angedeutet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

Die Anlage 100 weist einen Reaktor 10 auf, dem im dargestellten Beispiel ein Ethan enthaltendes, auf beliebige Weise gewonnenes Gasgemisch in Form eines Stoffstroms 101 zugeführt wird. Der Stoffstrom 101 kann beispielsweise einer nicht dargestellten Rektifikationseinheit entnommen werden, die höhere Kohlenwasserstoffe aus einem Ausgangsgemisch abtrennt. Der Stoffstrom 101 kann auch beispielsweise vorgewärmt und auf andere Weise aufbereitet werden. Der Stoffstrom 101 kann bereits Sauerstoff und ggf. ein Verdünnungsmittel wie Wasserdampf enthalten, entsprechende Medien können dem Reaktor jedoch auch, wie hier stellvertretend in Form von Stoffströmen 102 und 103 veranschaulicht, stromauf oder in dem Reaktor 10 zugegeben werden.

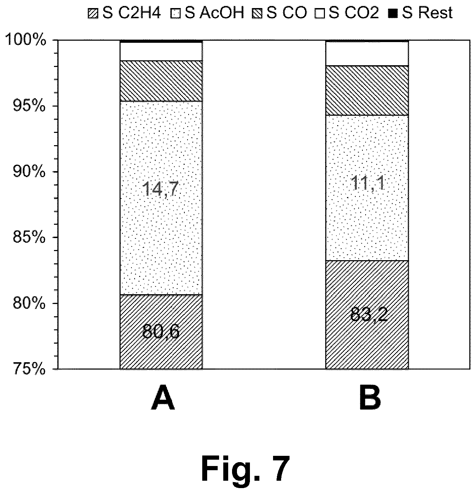

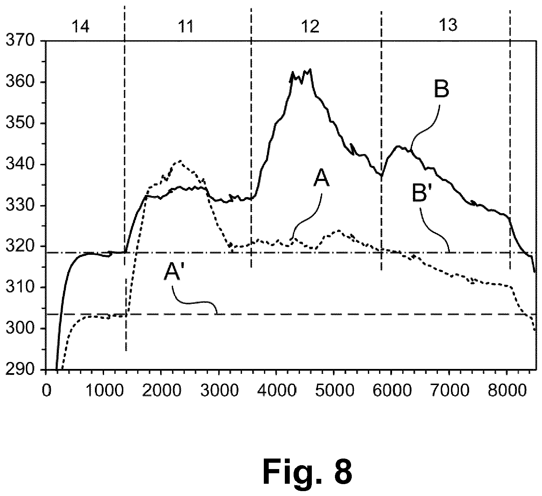

Der Reaktor 10 weist eine Vielzahl parallel angeordneter Reaktionsrohre 10c auf (nur zum Teil bezeichnet), die durch mehrere, im dargestellten Beispiel drei, Reaktionszonen 11, 12, 13 verlaufen, und die von einem Mantelbereich 10d umgeben sind. In den Reaktionsrohren 10c ist in den entsprechenden Reaktionszonen jeweils ein Katalysatorbett 11a, 12a, 13a vorgesehen (nur an einem Reaktionsrohr 10c veranschaulicht). Ein Ethan sowie Sauerstoff und ggf. ein Verdünnungsmittel enthaltendes Gasgemisch wird Form des Stoffstroms 101 bzw. der kombinierten Stoffströme 101 bis 103 nacheinander durch die Reaktionszonen 11, 12, 13 geführt. Den Reaktionszonen 11, 12, 13 ist eine inerte Zone 14 vorgeschaltet. Die Reaktionszonen 11, 12, 13 sind dabei zwischen einer Eintrittsöffnung 10a und einer Austrittsöffnung 10b des Reaktors 10 angeordnet, wobei eine der Reaktionszonen, hier die Reaktionszone 13, die näher an der Austrittsöffnung 10b angeordnet ist als eine andere der Reaktionszonen, hier eine der Reaktionszonen 11 und 12, als "zweite" Reaktionszone und eine der anderen Reaktionszonen 11, 12 als "erste" Reaktionszone bezeichnet wird. Das Katalysatorbett 13a der zweiten der Reaktionszone 13, durch die das Gasgemisch geführt wird, nachdem es zuvor durch die erste Reaktionszone 11, 12 geführt wurde, ist insbesondere mit einer höheren Katalysatorbeladung und/oder Katalysatoraktivität pro Raumeinheit ausgebildet als das Katalysatorbett 11a, 12a der ersten Reaktionszone 11, 12. Hierdurch kommt es zu den Vorteilen, die auch unter Bezugnahme auf die Figuren 7 und 8 nochmals erläutert werden. Alternativ oder zusätzlich kann auch eine zonal unterschiedliche Temperierung erfolgen.

Aus dem Reaktor 10 strömt ein Prozessgas in Form eines Prozessgasstroms 104 ab, in dem Ethylen enthalten ist, das in dem Reaktor 10 durch ODH eines Teils des Ethans in dem Reaktionseinsatzstrom gebildet wurde. Ferner enthält das Prozessgas Essigsäure, die ebenfalls bei der ODH in dem Reaktor 10 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in dem Reaktor 10 gebildet. Die Reaktionsrohre 10c werden mittels eines durch den Mantelbereichs geführten Temperiermittelstroms 105, 106 temperiert. Wie hier nicht veranschaulicht, können hierbei insbesondere mehrere Temperiermittelkreisläufe vorgesehen sein, die die Reaktionsrohre 10c abschnittsweise temperieren bzw. kühlen.

Es versteht sich, dass die Anlage 100 wie veranschaulicht einen, aber auch mehrere, beispielsweise parallel betriebene, Reaktoren 10 aufweisen kann. In letzterem Fall werden diesen Reaktoren 10 jeweils entsprechende Reaktionseinsätze, die gleich oder unterschiedlich zusammengesetzt sein können zugeführt, und es werden jeweils entsprechende Prozessgasströme 104 gebildet. Letztere können beispielsweise vereinigt und gemeinsam als Prozessgasnachfolgenden Verfahrensschritten bzw. Anlagenteilen zugeführt werden.

Stromab des Reaktors 10 kann ein Wasserpartialdruck erfasst werden. Dieser kann beispielsweise durch eine Zugabe von Wasser bzw. Dampf zu dem Gasgemisch des Stoffstroms 101 bzw. in Form der Stoffströme 102 oder 103 eingestellt werden. Eine weitere Beeinflussung, insbesondere eine Feineinstellung, kann durch Einstellen der Temperatur in dem Reaktor 100 erfolgen.

Nachfolgende Verfahrensschritte bzw. Anlagenkomponenten sind nicht veranschaulicht. Das Prozessgas kann in diesen mit Waschwasser oder einer geeigneten wässrigen Lösung in Kontakt gebracht werden, wodurch das Prozessgas insbesondere abgekühlt und Essigsäure aus dem Prozessgas ausgewaschen werden kann. Das zumindest weitgehend von Essigsäure befreite Prozessgas kann weiter aufbereitet und einer Abtrennung von Ethylen unterworfen werden. In dem Prozessgas enthaltendes Ethan kann in den Reaktor 10 zurückgeführt werden.

Figur 2 veranschaulicht in einem entsprechenden Verfahren erhaltene Selektivitäten zu Ethylen und Essigsäure in einem Diagramm, in dem Wasserpartialdrücke in bar (abs.) in einem aus einem Reaktor ausströmenden Prozessgas auf der Abszisse gegen Selektivitätswerte in Prozent auf der Ordinate aufgetragen sind. Die gezeigten Selektivitätswerte zu den einzelnen Produkten errechnen sich aus dem Verhältnis des jeweiligen Produktmolenstroms bezogen auf die molare Menge an Ethan, die pro Zeiteinheit im Reaktor umgesetzt wird.

Die gezeigten Daten beziehen sich auf zwei Versuchsreihen mit unterschiedlichen Durchströmraten, somit unterschiedlichen Raumgeschwindigkeiten und unterschiedlichen Temperaturen. Bei beiden Versuchsreihen wurde kein Ethylen am Reaktoreintritt hinzugegeben. Bei höheren Durchströmraten zeigen sich erwartungsgemäß niedrigere Umsätze (ca. 19% gegenüber ca. 40%), jedoch sind die Produktselektivitäten und somit das Produktmolenstromverhältnis (entspricht hier dem Verhältnis der beiden Selektivitäten) bei gleichen Wasserpartialdrücken am Reaktoraustritt nahezu identisch. Dies zeigt, dass die Prozesssteuerung in dem genannten Bereich maßgeblich auf den Wasserpartialdruck am Austritt gestützt werden kann.

Die bei den höheren Durchström- und geringeren Konversionsraten erhaltenen Werte sind für Ethylen mit ausgefüllten (schwarzen) Quadraten und für Essigsäure mit ausgefüllten (schwarzen) Dreiecken veranschaulicht, die bei den geringeren Durchström- und höheren Konversionsraten erhaltenen Werte entsprechend für Ethylen mit nicht ausgefüllten (weißen) Quadraten und für Essigsäure mit nicht ausgefüllten (weißen) Dreiecken.

Das Verhältnis der Produktmengen als Funktion des Wasserpartialdrucks am Reaktoraustritt ist nochmals in Figur 3 veranschaulicht. Hier sind die Wasserpartialdrücke in bar (abs.) auf der Abszisse gegen das Produktmolenstromverhältnis von Essigsäure zu Ethylen (entspricht hier dem Verhältnis der in Figur 2 gezeigten Werte zueinander) aufgetragen. Hier sind die Produktmolenstromverhältnisse für die höheren Durchström- und geringeren Konversionsraten mit ausgefüllten (schwarzen) Quadraten und für die geringeren Durchström- und höheren Konversionsraten mit nicht ausgefüllten (weißen) Quadraten veranschaulicht. Der teilweise klar lineare Verlauf des Produktmix wird vor allem für den wirtschaftlich relevanten Betrieb bei höheren Konversionen ersichtlich.

Dieses vereinfachte Verhalten des Reaktionssystems kann durch zwei Effekte erklärt werden, die experimentell nachgewiesen werden konnten, aber hier explizit als unverbindlich angegeben werden: Zum einen wird bei erhöhten Wasserpartialdrücken die Oxidation von gebildetem Ethylen begünstigt, wobei die Selektivität zur Bildung von Essigsäure zunimmt. Gleichzeitig wird die Desorption der gebildeten Essigsäure von der Katalysatoroberfläche durch erhöhte Wasserpartialdrücke begünstigt, wodurch weniger Essigsäure der ebenfalls am Katalysator stattfindenden nachfolgenden Oxidation von Essigsäure zu Kohlenmonoxid und Kohlendioxid zur Verfügung steht. Daher ergibt sich die Verschiebung der Gesamtselektivität hin zur Essigsäure, bei nahezu gleichbleibender Selektivität zu Kohlenmonoxid und Kohlendioxid.

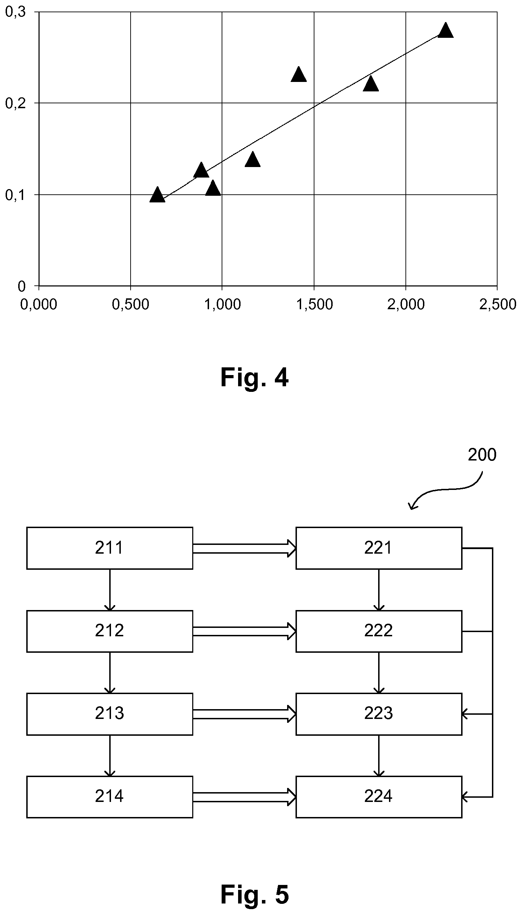

Der bestimmende Einfluss des Wasserpartialdrucks am Austritt auf das Produktverhältnis zwischen Essigsäure und Ethylen kann durch weitere Messungen belegt werden, zum Teil unter Verwendung unterschiedlicher Verdünnungsmedien und stark variierender Versuchsbedingungen. Hierzu wird auf Figur 4 verwiesen, die entsprechende Produktmolenstromverhältnisse von Essigsäure zu Ethylen zeigt. Die Darstellung entspricht jener der Figur 3.

Figur 5 veranschaulicht ein entsprechendes Verfahren in Form eines schematischen Ablaufplans, der insgesamt mit 200 bezeichnet ist. Mit 211 bis 214 sind jeweils zu erreichende Teilziele, mit 221 bis 224 die hierzu konkret vorzunehmenden Einstellungen bzw. Vorgaben bezeichnet.

Die gewünschte Produktverteilung von Essigsäure zu Ethylen wird in Schritt 211 vorgegeben. Auf dieser Grundlage wird in Schritt 221 ein Zielwert für den Wasserpartialdruck am Reaktoraustritt festgelegt. Auf Grundlage einer in Schritt 212 vorgegebenen Gesamtproduktmenge und zugehöriger Recyclemengen wird in Schritt 222 eine Durchflussmenge und damit die Konversion im Reaktor (siehe hierzu insbesondere Figuren 2 und 3) festgelegt.

In Schritt 213 wird ein entsprechend definierter Betriebspunkt angefahren, wozu in Schritt 223 ein Wasseranteil im Reaktionseinsatzstrom eingestellt wird. Die Feinabstimmung des Betriebspunkts, Schritt 214, erfolgt durch eine Anpassung der Reaktortemperatur in Schritt 224. Es wird jeweils der Wasserpartialdruck am Reaktoraustritt beobachtet.

In Figur 6 sind die Ergebnisse dreier ausgewählter Experimente 52, 56 und 71 veranschaulicht, die im Rahmen einer umfangreichen Versuchsreihe unter Verwendung eines Pilotreaktors durchgeführt wurden. Wiederum wurde im Rahmen der gesamten Versuchsreihe eine starke Korrelation des Produktverhältnisses von Ethylen zu Essigsäure zum Wasserpartialdruck am Austritt des Reaktors beobachtet. Dies gilt für unterschiedliche Konversionen und unterschiedliche Prozessbedingungen, d.h. veränderte Zusammensetzungen, Strommengen, Drücke und Temperaturen.

Die Experimente 52 und 71 wurden dabei bei gleichen Raumgeschwindigkeiten von 0,9 kg Ethan/(kg Katalysator × h) durchgeführt; im Experiment 56 betrug diese hingegen 1,4 kg Ethan/(kg Katalysator × h). Die Wasserpartialdrücke am Reaktoreintritt lagen für Experiment 52 bei 0,56 bar, für Experiment 56 bei 0,58 bar und für Experiment 71 bei 0,46 bar. Mit anderen Worten wurden in den Experimenten 52 und 56 nahezu identische Wasserpartialdrücke am Reaktoreintritt verwendet und bei Experiment 71 wich der Wasserpartialdruck am Reaktoreintritt deutlich ab. Die Wasserpartialdrücke am Reaktoraustritt lagen für Experiment 52 bei 1,28 bar, für Experiment 56 bei 0,99 bar und für Experiment 71 bei 1,00 bar. Mit anderen Worten wurden also in den Experimenten 56 und 71 nahezu identische Wasserpartialdrücke am Reaktoraustritt beobachtet und bei Experiment 52 wich der Wasserpartialdruck am Reaktoraustritt deutlich ab. Die unterschiedlichen Wasserpartialdrücke am Reaktoraustritt zwischen den Experimenten 52 und 56 ergaben sich aus den unterschiedlichen Raumgeschwindigkeiten bei im Wesentlichen gleichen Wasserpartialdrücken am Reaktoreintritt.

Die Versuchsbedingungen für die Experimente 52, 56 und 71 sind in der nachfolgenden Tabelle nochmals zusammengefasst. Die Salztemperatur stellt dabei die Temperatur einer Salzschmelze dar, die zur Kühlung des Reaktors verwendet wurde und bildet daher ein Maß für die Reaktortemperatur:

| **Experiment Nr.** | **52** | **56** | **71** |
|---|---|---|---|
| Reaktoreintrittsdruck [bar (abs.)] | 3,81 | 3,67 | 3,10 |
| Raumgeschwindigkeit [kg Ethan/(kg Katalysator × h)] | 0,9 | 1,4 | 0,9 |
| Wasser/Ethan [mol/mol] | 0,26 | | |
| Sauerstoff/Ethan [mol/mol] | 0,35 | 0,31 | 0,33 |
| Salztemperatur [°C] | 302 | 316 | 311 |
| Wasserpartialdruck Reaktoreintritt [bar (abs.)] | 0,56 | 0,58 | 0,46 |
| Wasserpartialdruck Reaktoraustritt [bar (abs.)] | 1,28 | 0,99 | 1,00 |

In Experiment 52 wurde ein Einsatz mit 56,7 Molprozent Ethan, 19,6 Molprozent Sauerstoff, 14,8 Molprozent Wasser und 8,9 Molprozent Stickstoff, in Experiment 56 ein Einsatz mit 60,2 Molprozent Ethan, 18,4 Molprozent Sauerstoff, 15,8 Molprozent Wasser und 5,7 Molprozent Stickstoff und in Experiment 71 wurde ein Einsatz mit 57,3 Molprozent Ethan, 18,8 Molprozent Sauerstoff, 14,9 Molprozent Wasser und 9,0 Molprozent Stickstoff verwendet.

In Figur 6 sind Werte zur Selektivität (S) für Ethylen (C2H4), Essigsäure (AcOH), Kohlenmonoxid (CO), Kohlendioxid (CO2) und restliche Verbindungen (Rest, aufgrund geringer Werte nicht sichtbar) für die drei Experimente 52, 56 und 71 veranschaulicht. Die Ordinate zeigt dabei die Werte bezüglich der Selektivitäten. Der Ethanumsatz variierte in den drei Experimenten 52, 56 und 71 um nicht mehr als 5%

Es zeigt sich deutlich, dass bei den Experimenten 56 und 71 ähnliche Produktverhältnisse bei ähnlichen Wasserpartialdrücken am Austritt bei unterschiedlichen Wasserpartialdrücken am Eintritt zu beobachten sind. Das Produktmolenstromverhältnis von Essigsäure zu Ethylen (entspricht hier dem Verhältnis der entsprechenden Selektivitäten) beträgt bei den Experimenten 56 und 71 jeweils rund 0,14. In den Experimenten 52 und 56 liegen hingegen ähnliche Wasserpartialdrücke am Eintritt, jedoch aufgrund der veränderten Raumgeschwindigkeiten deutlich unterschiedliche Wasserpartialdrücke am Austritt vor. Trotz ähnlicher Wasserpartialdrücke am Eintritt ergeben sich für die Versuchspunkte 52 und 56 auch deutlich unterschiedliche Produktverhältnisse. Das Produktmolenstromverhältnis von Essigsäure zu Ethylen beträgt rund 0,17 für Experiment 52 und liegt somit deutlich oberhalb des oben genannten Wertes für Experiment 56.

Im Rahmen der vorliegenden Erfindung kann insgesamt trotz erhöhter Umsatzraten im Vergleich zum Betrieb einem einlagigen Katalysatorbett bzw. einem Reaktor mit nur einer entsprechenden Reaktionszone eine Verschiebung der Wertproduktselektivität zu mehr Ethylen erreicht werden. Dies wird bei denselben Dampfverdünnungsraten im Reaktionseinsatz erzielt. Maßnahmen zur Steuerung der zeitlichen Entwicklung der Katalysatoraktivität durch Einstellen eines Wasserpartialdrucks im Reaktionseinsatz oder dem aus einem entsprechenden Reaktor abströmenden Gasgemisch behalten auch bei Verwendung eines mehrlagigen Betts ihre Gültigkeit.

Die gezeigten charakteristischen Selektivitätskurven können somit bei Verwendung eines adäquat ausgestalteten, mehrlagigen Katalysatorbetts bzw. einem Reaktor mit mehreren entsprechender Reaktionszonen parallel hin zu mehr Ethylen verschoben werden. Die Anpassungsmöglichkeiten im Betrieb auf Basis der Steuerung des Wasserpartialdrucks am Reaktoraustritt bleibt somit erhalten.

Die bei Verwendung eines einlagigen Betts beschriebenen Limitierungen in der weiteren wirtschaftlichen Optimierung des Prozesses können somit durch Anwendung einer Prozessführung mit mehrlagigen Betten und gezielter Temperatursteuerung überwunden werden. Die Wirtschaftlichkeit und die Vermarktungsfähigkeit der ODH-E Technologie verbessern sich somit spürbar.

In Figur 7 sind, vergleichbar mit Figur 6, Werte zur Selektivität (S) für Ethylen (C2H4), Essigsäure (AcOH), Kohlenmonoxid (CO), Kohlendioxid (CO2) und restliche Verbindungen (Rest, aufgrund geringer Werte auch nicht sichtbar) dargestellt, jedoch für den Fall A eines herkömmlichen Reaktors mit einlagigem Katalysatorbett und für den Fall B eines mehrlagigen Katalysatorbetts, in diesem Fall für einen Reaktor mit drei Reaktionszonen, die ansteigende Katalysatoraktivitäten bzw. Katalysatorgehalte pro Raumeinheit aufweisen. Die Ordinate zeigt auch hier die Werte bezüglich der Selektivitäten. Es wurde jeweils identische Zusammensetzungen des Reaktionseinsatzes und identische Massenströme verwendet.

In beiden Fällen A und B konnte keine nennenswerte Steigerung des Umsatzes durch weitere Temperaturerhöhung erreicht werden, ohne dass ein erhöhtes Risiko eines thermischen Durchgehens bzw. eine deutlich erhöhte Bildung von Kohlenstoffoxiden auftritt. Bei Verwendung eines dreilagigen Betts bzw. dreier entsprechender Reaktionszonen kann jedoch eine um 15 K höhere Mindesttemperatur in den jeweiligen Katalysatorzonen eingestellt werden, wodurch gemäß Fall B gegenüber Fall A eine deutliche Erhöhung von Umsatz und Ethylenselektivität erzielt werden kann. Die damit verbundenen Wertproduktverluste hin zu Kohlenstoffoxiden sind gering.

In 100% aller drei Reaktionszonen bzw. ihrer Katalysatorbetten wurden dabei Prozesstemperaturen auf der Mittelachse von mindestens 318,5°C eingehalten. In 100% der letzten zwei Reaktionszonen in Richtung des Reaktoraustritts (Fall B) werden sogar Prozesstemperaturen auf der Mittelachse von mindestens 327°C eingehalten. Im Vergleich dazu liegt die Mindesttemperatur im gesamten einlagigen Bett (Fall A) bei 303,5°C, am Ende des Katalysatorbetts bei 310°C.

In Figur 8 sind entsprechende Temperaturverläufe durch einen Reaktor 10 nochmals für die auch hier mit A und B bezeichneten Fälle veranschaulicht, wobei auf der Abszisse eine Reaktorlänge in mm und auf der Ordinate eine Temperatur in °C angegeben ist. Die auch hier mit 11, 12 und 13 bezeichneten Reaktionszonen sind nur in Fall B vorhanden. In Fall A ist anstelle der drei mit 11, 12 und 13 bezeichneten Reaktionszonen nur eine Reaktionszone vorhanden. In beiden Fällen liegt stromauf der Reaktionszone bzw. Reaktionszonen eine inerte Zone 14 vor. Ebenfalls veranschaulicht sind mit A' und B' bezeichnete Kühlmittel (Flüssigsalz-)Temperaturen.

## Patentansprüche

1. Verfahren zur Herstellung eines oder mehrerer Olefine und einer oder mehrerer Carbonsäuren, bei dem ein oder mehrerer Paraffine einer oxidativen Dehydrierung unterworfen wird oder werden, **dadurch gekennzeichnet, dass** für die oxidative Dehydrierung ein Reaktor (10) mit mehreren Reaktionszonen (11, 12, 13) verwendet wird, dass ein Gasgemisch mit dem einen oder den mehreren Paraffinen nacheinander durch die Reaktionszonen (11, 12, 13) geführt wird, und dass zumindest zwei der mehreren Reaktionszonen (11, 12, 13) einen Katalysator vom gleichen Katalysatortyp aufweisen und einer Temperaturbeeinflussung in unterschiedlichem Umfang unterworfen werden, wobei Katalysatoren vom gleichen Katalysatortyp Katalysatoren mit derselben Grundrezeptur sind, die in unterschiedlichen Konzentrationen oder Gehalten pro Raumeinheit bereitgestellt werden.

2. Verfahren nach Anspruch 1, bei dem in einer zweiten der Reaktionszonen (13), durch die das Gasgemisch geführt wird, nachdem es zuvor durch eine erste der Reaktionszonen (11, 12) geführt wurde, mit einer höheren Katalysatorbeladung und/oder mit einer höheren Katalysatoraktivität pro Raumeinheit ausgebildet wird als die erste Reaktionszone (11, 12).

3. Verfahren nach Anspruch 1 oder Anspruch 2, bei dem eine minimale und eine maximale Reaktionstemperatur vorgegeben werden und bei dem die Temperaturbeeinflussung in den Reaktionszonen (11, 12, 13) derart vorgenommen wird, dass in keiner der Reaktionszonen (11, 12, 13) an jeweils einer vorgegebenen Position die maximale Reaktionstemperatur überschritten und die minimale Reaktionstemperatur unterschritten wird.

4. Verfahren nach Anspruch 3, bei dem ein Reaktor (10) verwendet wird, der eine Anzahl zumindest teilweise parallel verlaufender Reaktionsrohre (10c) aufweist, wobei die vorgegebene Position auf der Mittelachse zumindest eines der mehreren Reaktionsrohre (10c) liegt.

5. Verfahren nach einem der vorstehenden Ansprüche, das derart durchgeführt wird, dass in mindestens 30% einer jeden der Reaktionszonen (11, 12, 13) die maximale Reaktionstemperatur nicht überschritten und die minimale Reaktionstemperatur nicht unterschritten wird.

6. Verfahren nach Anspruch 5, das derart durchgeführt wird, dass in der zweiten Reaktionszone (13) die maximale Reaktionstemperatur einem höheren Prozentanteil nicht überschritten und die minimale Reaktionstemperatur nicht unterschritten wird als in der ersten Reaktionszone (11, 12).

7. Verfahren nach einem der vorstehenden Ansprüche, bei dem der Reaktor (10) wenigstens eine weitere Reaktionszone (11 ) aufweist, durch die das Gasgemisch geführt wird, bevor es durch die erste Reaktionszone (12) und die zweite Reaktionszone (13) geführt wird.

8. Verfahren nach Anspruch 7, bei dem das Katalysatorbett (13a) der zweiten Reaktionszone (13) mit einer höheren Katalysatorbeladung und/oder Katalysatoraktivität pro Raumeinheit als das Katalysatorbett (11a) der weiteren Reaktionszone (11, 12) ausgebildet wird.

9. Verfahren nach einem der vorstehenden Ansprüche, bei dem Katalysatorbetten (11a, 12a, 13a) der Reaktionszonen (11, 12, 13) jeweils einen Anteil an aktivem Katalysator von mindestens 0,1 Gewichtsprozent aufweisen.

10. Verfahren nach einem der vorstehenden Ansprüche, bei dem die Reaktionszonen (11, 12, 13) mittels eines oder mehrerer Temperiermittelströme (105, 106) temperiert werden.

11. Verfahren nach Anspruch 10, bei dem ein Kühlsystem mit mehreren Temperiermittelströmen (105, 106) bereitgestellt wird, wobei zumindest einer der mehreren Temperiermittelströme (105, 106) zur Kühlung nur einer oder nur eines Teils der Reaktionszonen (11, 12, 13) verwendet wird.

12. Verfahren nach einem der vorstehenden Ansprüche 1, wobei dem Reaktor (10) ein wasserhaltiges Prozessgas entnommen wird und wobei das Verfahren umfasst, in dem dem Reaktor (10) entnommenen Prozessgas einen Wasserpartialdruck auf einen Wert in einem Bereich zwischen 0,5 und 5 bar (abs.) einzustellen.

13. Verfahren nach einem der vorstehenden Ansprüche, bei dem die Anzahl von Kohlenstoffatomen des Olefins, der Carbonsäure und des Paraffins zwei ist.

14. Anlage (100) zur Herstellung eines oder mehrerer Olefine und einer oder mehrerer Carbonsäuren, die dafür eingerichtet ist, ein oder mehrere Paraffine einer oxidativen Dehydrierung zu unterwerfen, **dadurch gekennzeichnet, dass** die Anlage (100) für die oxidative Dehydrierung einen Reaktor (10) aufweist, der mehrere Reaktionszonen (11, 12, 13) aufweist, dass Mittel bereitgestellt sind, die dafür eingerichtet sind, ein Gasgemisch mit dem einen oder den mehreren Paraffinen nacheinander durch die Reaktionszonen (11, 12, 13) zu führen, dass zumindest zwei der mehreren Reaktionszonen (11, 12, 13) einen Katalysator vom gleichen Katalysatortyp aufweisen, und dass Mittel bereitgestellt sind, die dafür eingerichtet sind, die zumindest zwei Reaktionszonen (11, 12, 13) einer Temperaturbeeinflussung in unterschiedlichem Umfang zu unterwerfen, wobei die Katalysatoren vom gleichen Katalysatortyp Katalysatoren mit derselben Grundrezeptur sind, die in unterschiedlichen Konzentrationen oder Gehalten pro Raumeinheit bereitgestellt werden.

## Claims

1. Method for producing one or more olefins and one or more carboxylic acids, in which one or more paraffins is or are subjected to oxidative dehydrogenation, **characterized in that** a reactor (10) having a plurality of reaction zones (11, 12, 13) is used for the oxidative dehydrogenation, **in that** a gas mixture comprising the one or more paraffins is successively passed through the reaction zones (11, 12, 13), **and in that** at least two of the plurality of reaction zones (11, 12, 13) have a catalyst of the same catalyst type and are subjected to varying temperature influences, catalysts of the same catalyst type being catalysts with the same basic formulation, which are provided in different concentrations or contents per unit volume.

2. Method according to claim 1, in which, in a second of the reaction zones (13) through which the gas mixture is passed after it has previously been passed through a first of the reaction zones (11 12), has a higher catalyst loading and/or a higher catalyst activity per unit volume than the first reaction zone (11, 12).

3. Method according to either claim 1 or claim 2, in which a minimum and a maximum reaction temperature are predetermined and in which the temperature influencing in the reaction zones (11, 12, 13) is carried out in such a way that, in any of the reaction zones (11, 12, 13) at any predetermined position, the maximum reaction temperature is not exceeded and the minimum reaction temperature does not fall short.

4. Method according to claim 3, in which a reactor (10) is used which comprises a number of at least partially parallel reaction tubes (10c), wherein the predetermined position lies on the central axis of at least one of the plurality of reaction tubes (10c).

5. Method according to any of the preceding claims, which is carried out in such a way that, in at least 30% of each of the reaction zones (11, 12, 13), the maximum reaction temperature is not exceeded and the minimum reaction temperature does not fall short.

6. Method according to claim 5, which is carried out in such a way that in the second reaction zone (13), the maximum reaction temperature is not exceeded a higher percentage and the minimum reaction temperature does not fall short than in the first reaction zone (11, 12).

7. Method according to any of the preceding claims, in which the reactor (10) has at least one further reaction zone (11) through which the gas mixture is passed before it is passed through the first reaction zone (12) and the second reaction zone (13).

8. Method according to claim 7, in which the catalyst bed (13a) of the second reaction zone (13) has a higher catalyst loading and/or catalyst activity per unit volume than the catalyst bed (11a) of the further reaction zone (11, 12).

9. Method according to any of the preceding claims, in which catalyst beds (11a, 12a, 13a) of the reaction zones (11, 12, 13) each have a proportion of active catalyst of at least 0.1 percent by weight.

10. Method according to any of the preceding claims, in which the reaction zones (11, 12, 13) are temperature controlled by means of one or more temperature control medium flows (105, 106).

11. Method according to claim 10, in which a cooling system is provided with a plurality of temperature control medium flows (105, 106), wherein at least one of the plurality of temperature control medium flows (105, 106) is used for cooling only one or only one part of the reaction zones (11, 12, 13).

12. Method according to any of the preceding claims 1, wherein a process gas containing water is removed from the reactor (10) and wherein the method comprises adjusting a water partial pressure in the process gas removed from the reactor (10) to a value in a range between 0.5 and 5 bar (abs.).

13. Method according to any of the preceding claims, in which the number of carbon atoms of the olefin, the carboxylic acid and the paraffin is two.

14. System (100) for producing one or more olefins and one or more carboxylic acids, which is designed to subject one or more paraffins to oxidative dehydrogenation, **characterized in that** the system (100) for the oxidative dehydrogenation has a reactor (10) comprising a plurality of reaction zones (11, 12, 13), **in that** means are provided that are designed to pass a gas mixture comprising the one or more paraffins successively through the reaction zones (11, 12, 13), **in that** at least two of the plurality of reaction zones (11, 12, 13) comprise a catalyst of the same catalyst type, **and in that** means are provided that are designed to subject the at least two reaction zones (11, 12, 13) to varying temperature influences, catalysts of the same catalyst type being catalysts with the same basic formulation, which are provided in different concentrations or contents per unit volume.

## Revendications

1. Procédé permettant la production d'une ou de plusieurs oléfines et d'un ou de plusieurs acides carboxyliques, dans lequel une ou plusieurs paraffines sont soumises à une déshydrogénation oxydante, **caractérisé en ce que,** pour la déshydrogénation oxydante, un réacteur (10) comportant plusieurs zones de réaction (11, 12, 13) est utilisé, **en ce qu'**un mélange gazeux comportant les une ou plusieurs paraffines est guidé séquentiellement à travers les zones de réaction (11, 12, 13), **et en ce qu'**au moins deux des plusieurs zones de réaction (11, 12, 13) présentent un catalyseur du même type de catalyseur et sont soumises à une influence de température à des degrés différents, dans lequel les catalyseurs du même type de catalyseur sont des catalyseurs comportant la même formule de base, lesquels catalyseurs sont fournis en différentes concentrations ou teneurs par unité de volume.

2. Procédé selon la revendication 1, dans lequel, dans une deuxième des zones de réaction (13), à travers laquelle le mélange gazeux est guidé après que celui-ci a préalablement été guidé à travers une première des zones de réaction (11, 12), est conçu avec une charge de catalyseur plus élevée et/ou avec une activité de catalyseur plus élevée par unité de volume que la première zone de réaction (11, 12).

3. Procédé selon la revendication 1 ou la revendication 2, dans lequel une température de réaction minimale et une température de réaction maximale sont prédéfinies et dans lequel l'influence de température dans les zones de réaction (11, 12, 13) est effectuée de telle sorte que, dans aucune des zones de réaction (11, 12, 13), à une position prédéfinie respective, la température de réaction maximale n'est dépassée et la température de réaction minimale n'est atteinte.

4. Procédé selon la revendication 3, dans lequel un réacteur (10) présentant un certain nombre de tubes de réaction (10c) au moins partiellement parallèles est utilisé, dans lequel la position prédéfinie est située sur l'axe central d'au moins l'un des plusieurs tubes de réaction (10c).

5. Procédé selon l'une des revendications précédentes, lequel est mis en oeuvre de telle sorte que, dans au moins 30 % de chacune des zones de réaction (11, 12, 13), la température de réaction maximale n'est pas dépassée et la température de réaction minimale n'est pas atteinte.

6. Procédé selon la revendication 5, lequel est mis en oeuvre de telle sorte que, dans la deuxième zone de réaction (13), la température de réaction maximale n'est pas dépassée d'un pourcentage plus élevé et la température de réaction minimale n'est pas dépassée vers le bas par rapport à dans la première zone de réaction (11, 12).

7. Procédé selon l'une des revendications précédentes, dans lequel le réacteur (10) présente au moins une autre zone de réaction (11) à travers laquelle le mélange de gaz est guidé avant que celui-ci ne soit guidé à travers la première zone de réaction (12) et la deuxième zone de réaction (13).

8. Procédé selon la revendication 7, dans lequel le lit de catalyseur (13a) de la deuxième zone de réaction (13) est conçu avec une charge de catalyseur et/ou une activité de catalyseur par unité de volume plus élevée que le lit de catalyseur (11a) de l'autre zone de réaction (11, 12).

9. Procédé selon l'une des revendications précédentes, dans lequel les lits de catalyseur (11a, 12a, 13a) des zones de réaction (11, 12, 13) présentent respectivement une proportion de catalyseur actif d'au moins 0,1 % en poids.

10. Procédé selon l'une des revendications précédentes, dans lequel les zones de réaction (11, 12, 13) sont tempérées à l'aide d'un ou de plusieurs flux de moyens de régulation de température (105, 106).

11. Procédé selon la revendication 10, dans lequel un système de refroidissement est fourni avec plusieurs flux de moyens de régulation de température (105, 106), dans lequel au moins l'un des plusieurs flux de moyens de régulation de température (105, 106) est utilisé pour refroidir uniquement une zone de réaction ou uniquement une partie des zones de réaction (11, 12, 13).

12. Procédé selon l'une des revendications précédentes 1, dans lequel un gaz de processus contenant de l'eau est prélevé dans le réacteur (10), et dans lequel le procédé comprend l'ajustement d'une pression partielle d'eau dans le gaz de processus prélevé dans le réacteur (10) à une valeur comprise dans une plage entre 0,5 et 5 bars (absolus).

13. Procédé selon l'une des revendications précédentes, dans lequel le nombre d'atomes de carbone de l'oléfine, de l'acide carboxylique et de la paraffine est de deux.

14. Installation (100) destinée à la production d'une ou de plusieurs oléfines et d'un ou de plusieurs acides carboxyliques, laquelle installation est conçue pour soumettre une ou plusieurs paraffines à une déshydrogénation oxydante, **caractérisée en ce que** l'installation (100) présente un réacteur (10) pour la déshydrogénation oxydante, lequel réacteur présente plusieurs zones de réaction (11, 12, 13), **en ce que** des moyens sont fournis, lesquels sont conçus pour permettre de guider un mélange gazeux comportant les une ou plusieurs paraffines séquentiellement à travers les zones de réaction (11, 12, 13), **en ce qu'**au moins deux des plusieurs zones de réaction (11, 12, 13) présentent un catalyseur du même type de catalyseur, **et en ce que** des moyens sont fournis, lesquels sont conçus pour soumettre les au moins deux zones de réaction (11, 12, 13) à une influence de température à des degrés différents, dans lequel les catalyseurs du même type de catalyseur sont des catalyseurs comportant la même formule de base, lesquels catalyseurs sont fournis en différentes concentrations ou teneurs par unité de volume.
